(19) Europäisches Patentamt
European Patent Office
Office européen des brevets

(11) **EP 3 870 957 B1**

(12) **EUROPEAN PATENT SPECIFICATION**

(45) Date of publication and mention
of the grant of the patent:
**16.07.2025 Bulletin 2025/29**

(21) Application number: **19848831.4**

(22) Date of filing: **23.10.2019**

(51) International Patent Classification (IPC):
***G01N 21/65*** (2006.01)    ***C12M 1/36*** (2006.01)
***G01N 21/84*** (2006.01)

(52) Cooperative Patent Classification (CPC):
**G01N 21/65; C12M 41/48; G01N 2021/8416;**
**G01N 2201/129**

(86) International application number:
**PCT/US2019/057513**

(87) International publication number:
**WO 2020/086635 (30.04.2020 Gazette 2020/18)**

(54) **AUTOMATIC CALIBRATION AND AUTOMATIC MAINTENANCE OF SPECTROSCOPIC MODELS FOR REAL-TIME PREDICTIONS**

AUTOMATISCHE KALIBRIERUNG UND AUTOMATISCHE AKTUALISIERUNG VON SPEKTROSKOPISCHEN MODELLEN FÜR ECHTZEIT-VORHERSAGEN

ÉTALONNAGE AUTOMATIQUE ET MAINTENANCE AUTOMATIQUE DES MODÈLES SPECTROSCOPIQUES POUR LES PRÉVISIONS EN TEMPS RÉEL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.10.2018 US 201862749359 P**
**12.04.2019 US 201962833044 P**
**21.06.2019 US 201962864565 P**

(43) Date of publication of application:
**01.09.2021 Bulletin 2021/35**

(73) Proprietor: **Amgen Inc.**
**Thousand Oaks, CA 91320-1799 (US)**

(72) Inventor: **TULSYAN, Aditya**
**Thousand Oaks, California 91320-1799 (US)**

(74) Representative: **Lau, Sarah Jane et al**
**Kilburn & Strode LLP**
**Lacon London**
**84 Theobalds Road**
**London WC1X 8NL (GB)**

(56) References cited:
**US-A1- 2018 291 329**

- **SU QING-LIN ET AL: "Just-in-Time-Learning based Extended Prediction Self-Adaptive Control for batch processes", JOURNAL OF PROCESS CONTROL, OXFORD, GB, vol. 43, 10 May 2016 (2016-05-10), pages 1 - 9, XP029553248, ISSN: 0959-1524, DOI: 10.1016/ J.JPROCONT.2016.04.009**
- **CHEN LINGYI ET AL: "A modified recursive locally weighted NIR modeling for fermentation process", 2017 6TH INTERNATIONAL SYMPOSIUM ON ADVANCED CONTROL OF INDUSTRIAL PROCESSES (ADCONIP), IEEE, 28 May 2017 (2017-05-28), pages 559 - 564, XP033126064, DOI: 10.1109/ ADCONIP.2017.7983841**
- **YI LIU ET AL: "Just-in-Time Kernel Learning with Adaptive Parameter Selection for Soft Sensor Modeling of Batch Processes", INDUSTRIAL & ENGINEERING CHEMISTRY RESEARCH, vol. 51, no. 11, 8 March 2012 (2012-03-08), pages 4313 - 4327, XP055673439, ISSN: 0888-5885, DOI: 10.1021/ie201650u**

EP 3 870 957 B1

- **ZHIQIANG GE ET AL: "A comparative study of just-in-time-learning based methods for online soft sensor modeling", CHEMOMETRICS AND INTELLIGENT LABORATORY SYSTEMS, vol. 104, no. 2, 25 September 2010 (2010-09-25), NL, pages 306 - 317, XP055673639, ISSN: 0169-7439, DOI: 10.1016/j.chemolab.2010.09.008**

- **GE ZHIQIANG ET AL: "Data Mining and Analytics in the Process Industry: The Role of Machine Learning", IEEE ACCESS, vol. 5, 26 September 2017 (2017-09-26), pages 20590 - 20616, XP011672147, DOI: 10.1109/ ACCESS.2017.2756872**

**Description**

**CROSS-REFERENCE TO RELATED APPLICATIONS**

Priority is claimed to U.S. Provisional Patent Application No. 62/749,359, filed October 23, 2018, U.S. Provisional Patent Application No. 62/833,044, filed April 12, 2019, and U.S. Provisional Patent Application No. 62/864,565, filed June 21, 2019.

**FIELD OF THE DISCLOSURE**

The present application relates generally to the monitoring and/or control of biopharmaceutical processes using spectroscopic techniques, such as Raman spectroscopy, and more specifically relates to the online calibration and maintenance of prediction models.

**BACKGROUND**

Stable production of biotherapeutic proteins by a biopharmaceutical process generally requires that a bioreactor maintain balanced and consistent parameters (e.g., cellular metabolic concentrations), which in turn demands rigorous process monitoring and control. To meet these demands, process analytical technology (PAT) tools are increasingly being adopted. Online monitoring of pH, dissolved oxygen, and cell culture temperature are a few examples of traditional PAT tools that have been used in feedback control systems. In recent years, other in-process probes have been investigated and deployed for continuous monitoring of more complex species, such as viable cell density (VCD), glucose, lactate, and other critical cellular metabolites, amino acids, titer, and critical quality attributes.

Raman spectroscopy is a popular PAT tool widely used for online monitoring in biomanufacturing. It is an optical method that enables non-destructive analysis of chemical composition and molecular structure. In Raman spectroscopy, incident laser light is scattered inelastically due to molecular vibration modes. The frequency difference between the incident and scattered photons is referred to as the "Raman shift," and the vector of Raman shift versus intensity levels (referred to herein as a "Raman spectrum," a "Raman scan," or a "Raman scan vector") can be analyzed to determine the chemical composition and molecular structure of a sample. Applications of Raman spectroscopy in polymer, pharmaceutical, biomanufacturing and biomedical analysis have surged in the past three decades as laser sampling and detector technology have improved. Due to these technological advances, Raman spectroscopy is now a practical analysis technique used both within and outside of the laboratory. Since the application of in-situ Raman measurements in biomanufacturing was first reported, it has been adopted to provide online, real-time predictions of several key process states, such as glucose, lactate, glutamate, glutamine, ammonia, VCD, and so on. These predictions are typically based on a calibration model or soft-sensor model that is built in an offline setting, based on analytical measurements from an analytical instrument. Partial least squares (PLS) and multiple linear regression modeling methods are commonly used to correlate the Raman spectra to the analytical measurements. These models typically require pre-processing filtering of the Raman scans prior to calibrating against the analytical measurements. Once a calibration model is trained, the model is implemented in a real-time setting to provide in-situ measurements for process monitoring and/or control.

Raman model calibration for biopharmaceutical applications is nontrivial, as biopharmaceutical processes typically operate under stringent constraints and regulations. The current state-of-the-art approach for Raman model calibration in the biopharmaceutical industry is to first run multiple campaign trials to generate relevant data that is used to correlate the Raman spectra to the analytical measurement(s). These trials are both expensive and time-consuming, as each campaign may last between two to four weeks in a laboratory setting, for example. Further, only limited samples may be available for the analytical instruments (e.g., to ensure that a lab-scale bioreactor maintains a healthy mass of viable cells). In fact, it is not uncommon to have only one or two measurements available each day from in-line or offline analytical instruments. To further exacerbate the situation, the current best practices yield calibration models that are tied to a specific process, the specific formula or profile of the bioreactor media, and the specific operating conditions. Thus, if any of the aforementioned variables were to change, the models may need to be re-calibrated based on new data. In fact, both Raman model calibration and model maintenance require significant resource allocations and are typically performed in an offline setting. While approaches that adapt models to new operating conditions have been proposed (e.g., recursive, moving-window, and time-difference methods), these methods may be unable to adequately handle abrupt process changes.

There are a number of publications describing generic Raman models based on traditional chemometric methods (e.g., PLS modeling) for multiple molecules. However, these generic models assume that the processes use similar, if not the same, media formulations and/or run process conditions. The media and processes are usually platformed with little or no variation. The drawback of this type of generic model is that once a process deviates from the norm, or if the training dataset contains too wide of a process range in an effort to account for the variations (e.g., media

**EP 3 870 957 B1**

additives, process duration and/or other process changes) between the different molecules, the generic models lose accuracy and precision. Therefore, these "generic" models are only generic within the described strict boundaries. See Mehdizaheh et al., Biotechnolo. Prog. 31(4): 1004-1013, 2015; Webster et al., Biotechnol. Prog. 34(3):730-737, 2018.

Su Qing-Lin et al: "Just-in-Time-Learning based Extended Prediction Self-Adaptive Control for batch processes", Journal of Process Control, Oxford, GB, vol. 43, 10 May 2016, pages 1-9 describes an Extended Prediction Self-Adaptive Control (EPSAC) algorithm based on the Just-in-Time Learning (JITL) method. In the proposed JITL-based EPSAC design, linearization of the process model is achieved by a set of local state-space models, each of which can be independently and simultaneously identified by the JITL method along the base trajectory.

Chen Lingyi et al: "A modified recursive locally weighted NIR modelling for fermentation process", 2017 6th International Symposium on Advanced Control of Industrial Processes (ADCONIP), IEEE, 28 May 2017, pages 559-564 describes a recursive modelling algorithm within a just-in-time framework by a moving window to deal with the time-varying and non-linear problems in near infrared (NIR) spectroscopy modelling. For a fermentation process, while moving window is adopted to facing the changing of target property, the initial database is expected to have a wide coverage to ensure the robustness of the NIR model (see abstract of D2). To balance between robustness (more initial samples included) and adaptability (more impact of new samples), a modified recursive locally weighted modelling approach is proposed and applied in a Chinese yellow wine (CYW) fermentation process. Meanwhile, the distance measurement of the original recursive locally weighted algorithm is improved by taking the target property into consideration.

Yi Liu et al: "Just-in-Time Kernel Learning with Adaptive Parameter Selection for Soft Sensor Modeling of Batch Processes", Industrial & Engineering Chemistry Research, vol. 51, no. 11, 8 March 2012, pages 4313-4327 describes an efficient nonlinear just-in-time learning (JITL) soft sensor method for online modeling of batch processes with uneven operating durations. A recursive least-squares support vector regression (RLSSVR) approach is combined with the JITL manner to model the nonlinearity of batch processes. The similarity between the query sample and the most relevant samples, including the weight of similarity and the size of the relevant set, can be chosen using a presented cumulative similarity factor. Then, the kernel parameters of the developed JITL-RLSSVR model structure can be determined adaptively using an efficient cross-validation strategy with low computational load. The soft sensor implement algorithm for batch processes is also developed. Both the batch-to-batch similarity and variation characteristics are taken into consideration to make the modeling procedure more practical. The superiority of the proposed soft sensor approach is demonstrated by predicting the concentrations of the active biomass and recombinant protein in the streptokinase fed-batch fermentation process, compared with other existing JITL-based and global soft sensors.

US 2018/291329 A1 relates, in some aspects, to the field of process analytical technology with respect to biotherapeutic protein production processes and products.

## BRIEF SUMMARY

The term "biopharmaceutical process" refers to a process used in biopharmaceutical manufacturing, such as a cell culture process to produce a desired recombinant protein. Cell culture takes place in a cell culture vessel, such as a bioreactor, under conditions that support the growth and maintenance of an organism engineered to express the protein. During recombinant protein production, process parameters, such as media component concentrations, including nutrients and metabolites (e.g., glucose, lactate, glutamate, glutamine, ammonia, amino acids, Na+, K+ and other nutrients or metabolites), media state (pH, pCO2, p02, temperature, osmolality, etc.), as well as cell and/or protein parameters (e.g., viable cell density (VCD), titer, cell state, critical quality attributes, etc.) are monitored for control and/or maintenance of the cell culture process.

To address some of the aforementioned limitations of the current best industrial practices, embodiments described herein relate to systems and methods that improve upon traditional techniques for spectroscopic analysis of biopharmaceutical processes, such as Raman spectroscopy. In particular, a "Just-In-Time Learning" (JITL) platform is used to build and maintain calibration models (e.g., Raman calibration models) in real-time for biopharmaceutical applications. JITL is a nonlinear modeling platform based on local modeling and database sampling technology. Unlike other machine-learning methods, JITL generally assumes that all available observations are stored in a central database, and models are dynamically built in real-time based upon a query, using the most relevant data from the database. This allows for good approximation of complicated process dynamics using relatively simple local models. Under the JITL framework, a library may contain spectral data not only for a single process operating under specific operating conditions, but also data for different processes, different media profiles, and/or different operation conditions. This can significantly reduce the time required to calibrate and maintain models, especially for pipeline drugs that may have little or no past production history.

The JITL platform maintains a dynamic library that may be updated each time a new analytical measurement is available. Further, to ensure that the local models adapt to new process conditions, the last available analytical measurement (e.g., for the product currently being monitored) may always be included in the training set for local modeling. This allows the local model to more quickly adapt to new conditions, or to new product lines with no history. Using

4

this approach, model calibration and model maintenance may both be automated, and the time and expense (e.g., material and labor costs) associated with routine calibrations in conventional systems may be greatly reduced. Moreover, the ability to provide credibility bounds (or other confidence indicators, such as confidence scores) around model predictions may allow for robust monitoring and control strategies.

According to the invention, Gaussian process models are used for local modeling, within the JITL framework. Gaussian process models are powerful statistical machine-learning models that can efficiently capture complex nonlinear process dynamics, and can readily adapt to virtually any process changes. In contrast to PLS, principal component regression (PCR) and other types of regression models, Gaussian process models are non-parametric methods, and are far more capable of capturing complex correlations between the Raman spectra and the analytical measurements from limited data sets. Moreover, Gaussian process models generally do not require pre-processing filtering of the Raman scans. Accordingly, in some embodiments, the Gaussian process models are instead calibrated on the raw Raman scans (in logarithmic scale), which may save many steps in the model calibration/maintenance process. Furthermore, Gaussian process models provide credibility bounds around the predictions, which can be extremely difficult to obtain using PLS or PCR models. Credibility bounds can be particularly useful for designing optimal sampling strategies for analytical instruments, and/or for implementing closed-loop control (e.g., model-predictive control, or MPC), for instance, to avoid making changes based on unreliable predictions.

Although JITL is a nonlinear modeling framework, and although the approach described above provides some adaptability by updating the dynamic library with recent analytical measurements, JITL alone may not be sufficiently adaptive to account for time-varying process conditions (e.g., abrupt changes to the set-point or other process conditions). In particular, local models that are calibrated using JITL may fail to make use of recent samples. For example, and particularly if there has been a recent and abrupt change in process conditions, the recent samples may fail to satisfy a similarity criterion that is based purely on "spatial" similarity (e.g., similarity of the Raman scans). Modified JITL techniques that can better leverage the information offered by recent samples (irrespective of spatial similarity), and therefore can better adapt to time-varying process changes, are also described herein. In particular, "adaptive" JITL (A-JITL) and "spatiotemporal" JITL (ST-JITL) techniques for model calibration and maintenance are described herein.

Real-time model maintenance, in which local models can learn from the latest analytical measurements and thereby adapt quickly to time-varying conditions, can be important to the success of JITL techniques. However, frequent access to analytical instruments/measurements (e.g., analyzing offline samples) tends to be highly resource-intensive. To minimize such resource usage, without overly degrading model performance, a performance-based model maintenance protocol may be implemented in which the system schedules/triggers an analytical measurement in response to determining that the current model performance is unacceptable/unreliable.

## BRIEF DESCRIPTION OF THE DRAWINGS

The skilled artisan will understand that the figures, described herein, are included for purposes of illustration and are not limiting on the present disclosure. The drawings are not necessarily to scale, emphasis instead being placed upon illustrating the principles of the present disclosure. It is to be understood that, in some instances, various aspects of the described implementations may be shown exaggerated or enlarged to facilitate an understanding of the described implementations. In the drawings, like reference characters throughout the various drawings generally refer to functionally similar and/or structurally similar components.

FIG. 1 is a simplified block diagram of an example Raman spectroscopy system that may be used to predict analytical measurements of biopharmaceutical processes.

FIG. 2 is a simplified block diagram of an example Raman spectroscopy system that may be used to predict analytical measurements of biopharmaceutical processes for closed-loop control of glucose concentration.

FIG. 3 depicts experimental results for closed-loop control of glucose concentration using an example implementation of the Raman spectroscopy system described herein.

FIG. 4 depicts an example data flow that may occur when analyzing a biopharmaceutical process using a Just-In-Time Learning (JITL) technique.

FIG. 5 depicts an example data flow that may occur when analyzing a biopharmaceutical process using an adaptive JITL (A-JITL) technique.

FIG. 6 depicts an example data flow that may occur when analyzing a biopharmaceutical process using a spatiotemporal JITL (ST-JITL) technique.

FIG. 7 is a flow diagram of an example method for analyzing a biopharmaceutical process.

## DETAILED DESCRIPTION

The various concepts introduced above and discussed in greater detail below may be implemented in any of numerous ways, and the described concepts are not limited to any particular manner of implementation. Examples of implementations are provided for illustrative purposes.

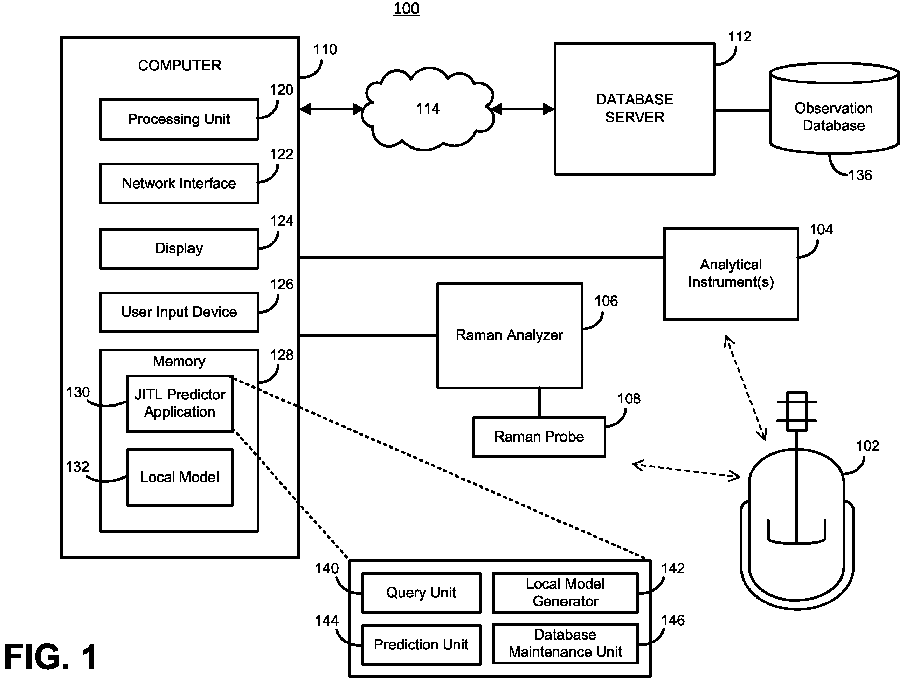

FIG. 1 is a simplified block diagram of an example Raman spectroscopy system 100 that may be used to predict analytical measurements of biopharmaceutical processes. While FIG. 1 depicts a system 100 that implements Raman spectroscopy techniques, it is understood that, in other embodiments, system 100 may implement other spectroscopy techniques suitable for analyzing biopharmaceutical processes, such as near-infrared (NIR) spectroscopy, for example.

System 100 includes a bioreactor 102, one or more analytical instruments 104, a Raman analyzer 106 with Raman probe 108, a computer 110, and a database server 112 that is coupled to computer 110 via a network 114. Bioreactor 102 may be any suitable vessel, device or system that supports a biologically active environment, which may include living organisms and/or substances derived therefrom (e.g., a cell culture) within a media. Bioreactor 102 may contain recombinant proteins that are being expressed by the cell culture, e.g., such as for research purposes, clinical use, commercial sale or other distribution. Depending on the biopharmaceutical process being monitored, the media may include a particular fluid (e.g., a "broth") and specific nutrients, and may have target media state parameters, such as a target pH level or range, a target temperature or temperature range, and so on. The media may also include organisms and substances derived from the organisms such as metabolites and recombinant proteins. Collectively, the contents and parameters/characteristics of media are referred to herein as the "media profile."

Analytical instrument(s) 104 may be any in-line, at-line and/or offline instrument, or instruments, configured to measure one or more characteristics or parameters of the biologically active contents within bioreactor 102, based on samples taken therefrom. For example, analytical instrument(s) 104 may measure one or more media component concentrations, such as nutrient and/or metabolite levels (e.g., glucose, lactate, glutamate, glutamine, ammonia, amino acids, Na+, K+, etc.) and media state parameters (pH, $pCO_2$, $pO_2$, temperature, osmolality, etc.). Additionally, or alternatively, analytical instrument(s) 104 may measure osmolality, viable cell density (VCD), titer, critical quality attributes, cell state (e.g., cell cycle) and/or other characteristics or parameters associated with the contents of bioreactor 102. As a more specific example, samples may be taken, spun down, purified by multiple columns, and run through a first one of analytical instruments 104 (e.g., a high performance liquid chromatography (HPLC) or ultra high performance liquid chromatograpy (UPLC) instrument), followed by a second one of analytical instruments 104 (e.g., a mass spectrometer), with both the first and second analytical instruments 104 providing analytical measurements. One, some or all of analytical instrument(s) 104 may use destructive analysis techniques.

Raman analyzer 106 may include a spectrograph device coupled to Raman probe 108 (or, in some implementations, multiple Raman probes). Raman analyzer 106 may include a laser light source that delivers the laser light to Raman probe 108 via a fiber optic cable, and may also include a charge-coupled device (CCD) or other suitable camera/recording device to record signals that are received from Raman probe 108 via another channel of the fiber optic cable, for example. Alternatively, the laser light source may be integrated within Raman probe 108 itself. Raman probe 108 may be an immersion probe, or any other suitable type of probe (e.g., a reflectance probe and transmission probe).

Collectively, Raman analyzer 106 and Raman probe 108 are configured to non-destructively scan the biologically active contents during the biopharmaceutical process within bioreactor 102 by exciting, observing, and recording a molecular "fingerprint" of the biopharmaceutical process. The molecular fingerprint corresponds to the vibrational, rotational and/or other low-frequency modes of molecules within the biologically active contents within the biopharmaceutical process when the bioreactor contents are excited by the laser light delivered by Raman probe 108. As a result of this scanning process, Raman analyzer 106 generates one or more Raman scan vectors that each represent intensity as a function of Raman shift (frequency).

Computer 110 is coupled to Raman analyzer 106 and analytical instrument(s) 104, and is generally configured to analyze the Raman scan vectors generated by Raman analyzer 106 in order to predict one or more analytical measurements of the biopharmaceutical process. For example, computer 110 may analyze the Raman scan vectors to predict the same type(s) of analytical measurement(s) that are made by analytical instrument(s) 104. As a more specific example, computer 110 may predict glucose concentrations, while analytical instrument(s) 104 actually measure glucose concentrations. However, whereas analytical instrument(s) 104 may make relatively infrequent, "offline" analytical measurements of samples extracted from bioreactor 102 (e.g., due to limited quantities of the media from the biopharmaceutical process, and/or due to the higher cost of making such measurements, etc.), computer 110 may make relatively frequent, "online" predictions of analytical measurements in real-time. Computer 110 may also be configured to transmit analytical measurements made by analytical instrument(s) 104 to database server 112 via network 114, as will be discussed in further detail below.

In the example embodiment shown in FIG. 1, computer 110 includes a processing unit 120, a network interface 122, a display 124, a user input device 126, and a memory 128. Processing unit 120 includes one or more processors, each of which may be a programmable microprocessor that executes software instructions stored in memory 128 to execute some or all of the functions of computer 110 as described herein. Alternatively, one, some or all of the processors in processing unit 120 may be other types of processors (e.g., application-specific integrated circuits (ASICs), field-programmable gate arrays (FPGAs), etc.), and the functionality of computer 110 as described herein may instead be implemented, in part or in whole, in hardware. Memory 128 may include one or more physical memory devices or units containing volatile and/or non-volatile memory. Any suitable memory type or types may be used, such as read-only memory (ROM), solid-state drives (SSDs), hard disk drives (HDDs), and so on.

Network interface 122 may include any suitable hardware (e.g., front-end transmitter and receiver hardware), firmware, and/or software configured to communicate via network 114 using one or more communication protocols. For example, network interface 122 may be or include an Ethernet interface. Network 114 may be a single communication network, or may include multiple communication networks of one or more types (e.g., one or more wired and/or wireless local area networks (LANs), and/or one or more wired and/or wireless wide area networks (WANs) such as the Internet or an intranet, for example).

Display 124 may use any suitable display technology (e.g., LED, OLED, LCD, etc.) to present information to a user, and user input device 126 may be a keyboard or other suitable input device. In some embodiments, display 124 and user input device 126 are integrated within a single device (e.g., a touchscreen display). Generally, display 124 and user input device 126 may combine to enable a user to interact with graphical user interfaces (GUIs) provided by computer 110, e.g., for purposes such as manually monitoring various processes being executed within system 100. In some embodiments, however, computer 110 does not include display 124 and/or user input device 126, or one or both of display 124 and user input device 126 are included in another computer or system that is communicatively coupled to computer 110 (e.g., in some embodiments where predictions are sent directly to a control system that implements closed-loop control).

Memory 128 stores the instructions of one or more software applications, including a Just-In-Time-Learning (JITL) predictor application 130. JITL predictor application 130, when executed by processing unit 120, is generally configured to predict analytical measurements of the biopharmaceutical process in bioreactor 102 by calibrating a local model 132, and by using local model 132 to analyze Raman scan vectors generated by Raman analyzer 106. Depending on the frequency at which Raman analyzer 106 generates such scan vectors, JITL predictor application 130 may predict analytical measurements on a periodic or other suitable time basis. Raman analyzer 106 may itself control when scan vectors are generated, or computer 110 may trigger the generation of scan vectors by sending a command to Raman analyzer 106. JITL predictor application 130 may predict only a single type of analytical measurement based on each scan vector (e.g., only glucose concentration), or may predict multiple types of analytical measurements based on each scan vector (e.g., glucose concentration and viable cell density). In other embodiments, multiple different JITL predictor applications (e.g., each similar to JITL predictor application 130) each generate a different local model to predict a different type of analytical measurement, all based on the same scan vector. JITL predictor application 130 and local model 132 will be discussed in further detail below.

Database server 112 may be remote from computer 110 (e.g., such that a local setup may include only bioreactor 102, analytical instrument(s) 104, Raman analyzer 106 with Raman probe 108, and computer 110) and, as seen in FIG. 1, may contain or be communicatively coupled to an observation database 136 that stores observation data sets associated with past observations. Each observation data set in observation database 136 may include spectral data (e.g., one or more Raman scan vectors of the sort produced by Raman analyzer 106) and one or more corresponding analytical measurements (e.g., one or more measurements of the sort(s) produced by analytical instrument(s) 104). Depending on the embodiment and/or scenario, the past observations may have been collected for a number of different biopharmaceutical processes, under a number of different operation conditions (e.g., different metabolite concentration set points), and/or with a number of different media profiles (e.g., different fluids, nutrients, pH levels, temperatures, etc.). Generally, it may be desirable to have observation database 136 represent a broadly diverse array of processes, operating conditions, and media profiles. Observation database 136 may or may not store information indicative of those processes, cell lines, proteins, metabolites, operating conditions, and/or media profiles, however, depending on the embodiment (as discussed further below). In some embodiments, database server 112 is remotely coupled to multiple other computers similar to computer 110, via network 114 and/or other networks. This may be desirable in order to collect a larger number of observation data sets for storage in observation database 136. In other embodiments, however, system 100 does not include database server 112, and computer 110 directly accesses a local observation database 136.

It is understood that other configurations and/or components may be used instead of those shown in FIG. 1. For example, a different computer (not shown in FIG. 1) may transmit measurements provided by analytical instrument(s) 104 to database server 112, one or more additional computing devices or systems may act as intermediaries between computer 110 and database server 112, some or all of the functionality of computer 110 as described herein may instead be performed remotely by database server 112 and/or another remote server, and so on.

During run-time operation of system 100, Raman analyzer 106 and Raman probe 108 are used to scan (i.e., generate Raman scan vectors for) a biopharmaceutical process in bioreactor 102, and the Raman scan vector(s) is/are then transmitted from Raman analyzer 106 to computer 110. Raman analyzer 106 and Raman probe 108 may provide scan vectors to support predictions (made by JITL predictor application 130) according to a predetermined schedule of monitoring periods, such as once per minute, or once per hour, etc. Alternatively, predictions may be made at irregular intervals (e.g., in response to a certain process-based trigger, such as a change in measured pH level and/or temperature), such that each monitoring period has a variable or uncertain duration. Depending on the embodiment, Raman analyzer 106 may send only one scan vector to computer 110 per monitoring period, or multiple scan vectors to computer 110 per monitoring period, depending on how many scan vectors local model 132 accepts as input for a single prediction. Multiple scan vectors may improve the prediction accuracy of local model 132, for example.

A query unit 140 of JITL predictor application 130 uses the scan vector(s) received for a single monitoring period to generate a query point that will be used to query observation database 136. In some embodiments, the query point (i.e., the data defining the query point) includes only data representing the Raman scan vector(s) that was/were received from Raman analyzer 106 (e.g., intensity/frequency tuples that comprise each scan vector). In other embodiments, the query point also includes one or more other types information. For example, the query point may also include data representing operating conditions associated with the process (e.g., a metabolite concentration set point in a control system, or a laser light wavelength and/or intensity associated with Raman analyzer 106 or Raman probe 108, etc.), data representing the media profile for the biopharmaceutical process media (e.g., fluid type, nutrient types or concentrations, pH level, etc.), and/or other data (e.g., indicators of cell lines, proteins or metabolites associated with the biopharmaceutical process).

Generally, the query point may include data representing the same vectors, parameters, and/or classifications that local model 132 uses as inputs (i.e., as the feature set of local model 132). Use of a number of different data types for the feature set may improve accuracy of the analytical measurement predictions made by local model 132. However, because each observation data set in observation database 136 would generally need to include the same vectors, parameters, and/or classifications as the feature set, it may be preferable to limit the query point, and the feature set/inputs of local model 132, to only include one or more Raman scan vector(s). This may provide various benefits, such as allowing the collection of more information for storage in observation database 136, and/or simplifying the collection of that information. If only Raman scan vectors are used, for example, observation data sets may be included in observation database 136 even if little or nothing is known about the processes, cell lines, proteins, metabolites, operating conditions, and/or media profiles that existed when the data sets were collected.

Query unit 140 then queries observation database 136 using the generated query point. In the example embodiment of FIG. 1, query unit 140 accomplishes this by causing network interface 122 to transmit the query point (e.g., within a query message) to database server 112 via network 114, which in turn causes database server 112 to retrieve the appropriate data from observation database 136. In embodiments where observation database 136 is instead included in (or in a memory communicatively coupled to) computer 110, however, query unit 140 may instead query observation database 136 more directly. For ease of explanation, the remaining description of FIG. 1 will assume that observation database 136 is coupled to database server 112, as depicted in FIG. 1. However, one of ordinary skill in the art will readily understand how the communication paths may differ if observation database 136 were instead local to computer 110, or in another suitable location within a system architecture.

After receiving the query point, database server 112 uses the query point to select relevant observation data sets from observation database 136 that will be useful as training data for local model 132. Database server 112 may apply any suitable relevancy criteria to identify which observation data sets are "relevant," depending on the embodiment. In one embodiment, for example, the query point includes a single Raman scan vector, and database server 112 determines whether a given observation data set is relevant by calculating a Euclidean distance between the Raman scan vector of that observation data set and the Raman scan vector of the query point. If the Euclidean distance is below some predetermined threshold value (or below a variable threshold, such as a threshold calculated based on the average Euclidean distance between the query point scan vector and all observation data set scan vectors, etc.), the observation data set is identified as a relevant observation data set. One of ordinary skill in the art will understand how such an approach could easily be extended to embodiments in which the query point (and each observation data set) includes multiple Raman scan vectors. In some situations, use of Euclidean distance to select relevant observation data sets may be a sub-optimal technique. As the local model 132 is a Gaussian process model (as discussed below), use of Euclidean distance as a relevancy criterion may be particularly advantageous. This is because Gaussian process models with radial-basis functions or squared-exponential kernels are themselves based on Euclidean distance. Nonetheless, in other embodiments, other relevancy criteria may be applied (e.g., angle-based or correlation-based criteria, etc.). It is understood that, in embodiments where local model 132 also accepts other information as an input/feature set (e.g., operating conditions, media profile, process data, cell line information, protein information, and/or metabolite information, etc.), more complex techniques may be used to identify "relevant" observation data sets. In some embodiments, database server 112 selects only a predetermined number of relevant observation data sets in response to a single query, or selects no more than some maximum allowed number of relevant observation data sets, to ensure that only a relatively small subset of all datasets within observation database 136 is retrieved. In other embodiments, however, database server 112 can select any number of relevant observation data sets, so long as the relevancy criteria are satisfied for each such data set.

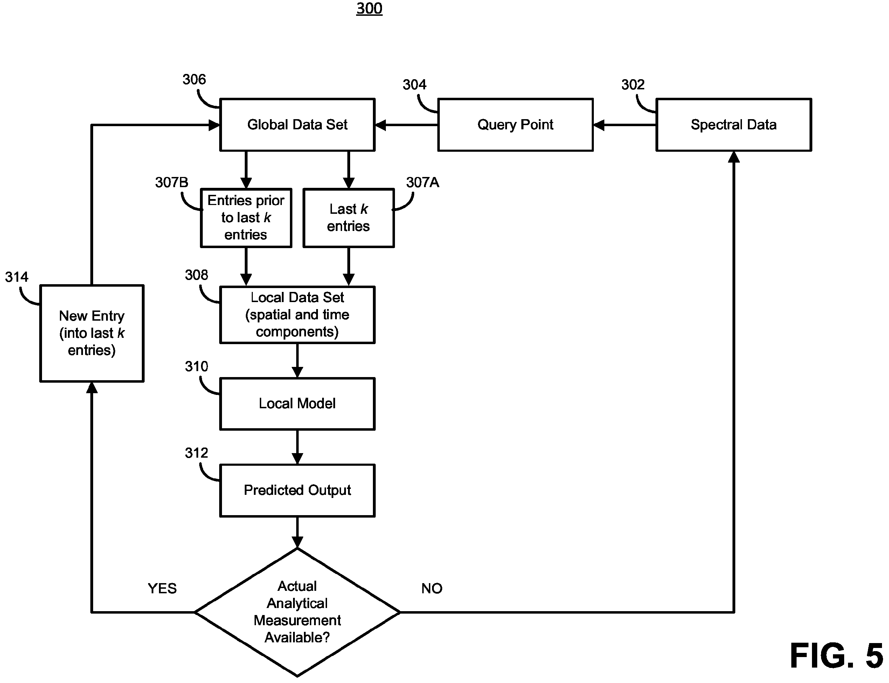

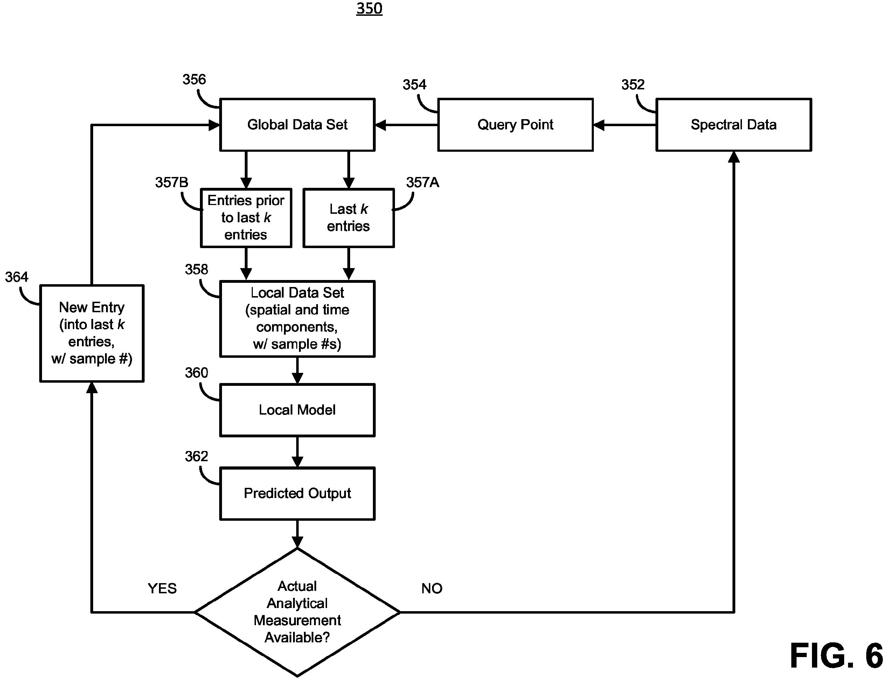

In some embodiments, as will be described in more detail below (e.g., with reference to FIGs. 5 and 6), the relevant observation data sets are selected based not only on relevance to a query point in a "spatial" sense (e.g., similarity of Raman scan vectors), but also on relevance in a temporal sense (e.g., which data sets are most recent, regardless of spatial similarity). These techniques may better leverage the fact that more recent analytical measurements can provide useful information, even when those recent measurements correspond to a different set-point, etc.

After identifying the relevant observation data sets (each of which may or may not correspond to the same process conditions as the biopharmaceutical process in bioreactor 102 that is currently being monitored), database server 112 retrieves those data sets (e.g., the Raman scan vectors and corresponding analytical measurement(s)), and transmits the retrieved data sets to computer 110 via network 114. Query unit 140 may then pass the relevant data sets to local model generator 142, and local model generator 142 uses the relevant data sets as training data to calibrate local model 132. That is, local model generator 142 uses the Raman scan vector(s) (and possibly other data) associated with each observation data set as a feature set, and uses the analytical measurement(s) associated with the same observation data set as a label for that feature set.

According to the invention, as noted above, local model generator 142 builds a Gaussian process model in order to efficiently capture complex, nonlinear process dynamics, and to readily adapt to virtually any process changes. Unlike PLS and PCR models, Gaussian process models use non-parametric methods, and are far more capable of capturing complex nonlinear correlations between the Raman scan vectors and the analytical measurements, even when using a very limited number of training samples. This can be particularly important in scenarios where new products or processes correspond to only a limited number of data sets in observation database 136. In such scenarios, a Gaussian process model is generally able to extract the most information from those limited data sets, in conjunction with the other relevant data sets that database server 112 selects from observation database 136. In other embodiments, however, local model generator 142 may instead build any other suitable type of machine-learning model (e.g., a recursive neural network, a convolutional neural network, etc.), so long as the training time does not exceed the minimum desired duration of a monitoring period. Local model generator 142 may also build local model 132 such that local model 132 can output credibility bounds, or some other suitable indicator of prediction confidence (e.g., a confidence score). At least as compared to PLS and PCR models, Gaussian process models are particularly well-suited for providing credibility bounds around the analytical measurement predictions. While various advantages of Gaussian process models over PLS and PCR models have been described, it is understood that, in some embodiments, local model generator 142 may use PLS or PCR modeling methods to build local model 132.

Local model generator 142 may build local model 132 in an online, real-time manner, such that prediction unit 144 can then use the trained local model 132 to predict one or more analytical measurements of the biopharmaceutical process by processing the same Raman scan vector(s) that query unit 140 had used to generate the query point. Indeed, in some embodiments, query unit 140 may perform a new query, and local model generator 142 may generate a new version of local model 132, each and every time that Raman analyzer 106 provides a new Raman scan vector (or a new set of Raman scan vectors) to computer 110. In other embodiments, however, query unit 140 performs a new query (and local model generator 142 generates a new version of local model 132) on a less frequent basis, such as once every 10 predictions/monitoring periods, or once every 100 predictions/monitoring periods, etc.

Database maintenance unit 146 may also cause analytical instrument(s) 104 to periodically collect one or more actual analytical measurements, at a significantly lower frequency than the monitoring period of Raman analyzer 106 (e.g., only once or twice per day, etc.). The measurement(s) by analytical instrument(s) 104 may be destructive, in some embodiments, and require permanently removing a sample from the process in bioreactor 102. At or near the time that database maintenance unit 146 causes analytical instrument(s) 104 to collect and provide the actual analytical measurement(s), database maintenance unit 146 may also cause Raman analyzer 106 to provide one or more Raman scan vectors. Database maintenance unit 146 may then cause network interface 122 to send the Raman scan vector(s) and corresponding actual analytical measurement(s) to database server 112 via network 114, for storage as a new observation data set in observation database 136. Observation database 132 may be updated according to any suitable timing, which may vary depending on the embodiment. If analytical instrument(s) 104 output(s) actual analytical measurements within seconds of measuring a sample, for instance, observation database 132 may be updated with new measurements almost immediately as samples are taken. In certain other embodiments, however, the actual analytical measurements may be the result of minutes, hours or even days of processing by one or more of analytical instrument(s) 104, in which case observation database 132 is not updated until after such processing has been completed. In still other embodiments, new observation datasets may be added to observation database 132 in an incremental manner, as different ones of analytical instruments 104 complete their respective measurements.

Thus, observation database 136 provides a "dynamic library" of past observations that local model generator 142 may draw upon for model training. In some embodiments, the latest analytical measurement(s) is/are always added to observation database 136, and local model generator 142 may always use the most recent observation data set(s) in observation database 136 when calibrating local model 132. This may allow local model 132 to encode the process information from the recent past and to quickly adapt to new conditions, or quickly adapt to new process conditions with no history. Moreover, both calibration and maintenance of local model 132 may be automated. In some embodiments, adaptability of the local model 132 is further enhanced, e.g., as discussed below in connection with the A-JITL and ST-JITL techniques.

In some embodiments, database maintenance unit 146 may cause analytical instrument(s) 104 to collect and provide the actual analytical measurement(s) on some other time basis or condition, such as current model performance.

According to the invention, the local model 132 outputs a credibility interval (e.g., the range of values, around the predicted value, within which there is a 95% probability or confidence that an actual/measured value would fall) or some other confidence indicator along with a prediction (local model 132 is a Gaussian process model), and if the confidence indicator reveals a particularly unreliable prediction (e.g., if the interval/range exceeds a threshold width/range, etc.), then database maintenance unit 146 may trigger the collection of one or more actual analytical measurements. As a more specific example, database maintenance unit 146 may trigger the collection of the analytical measurement(s) in response to determining that a 95% credibility interval exceeds a pre-defined threshold. Optimal scheduling of analytical measurements is discussed in further detail below. After the measurement(s) is/are made, database maintenance unit 146 may cause Raman analyzer 106 to generate one or more Raman scan vectors, and cause network interface 122 to provide the actual analytical measurement(s) and the corresponding Raman scan vector(s) to database server 112 for storage as a new observation data set in observation database 132 (e.g., in the manner discussed above). Local model generator 142 may then utilize that latest observation data set, if appropriate (e.g., depending on the relevance to the current query, or whether the embodiment always makes use of the most recent observation data set), when calibrating local model 132.

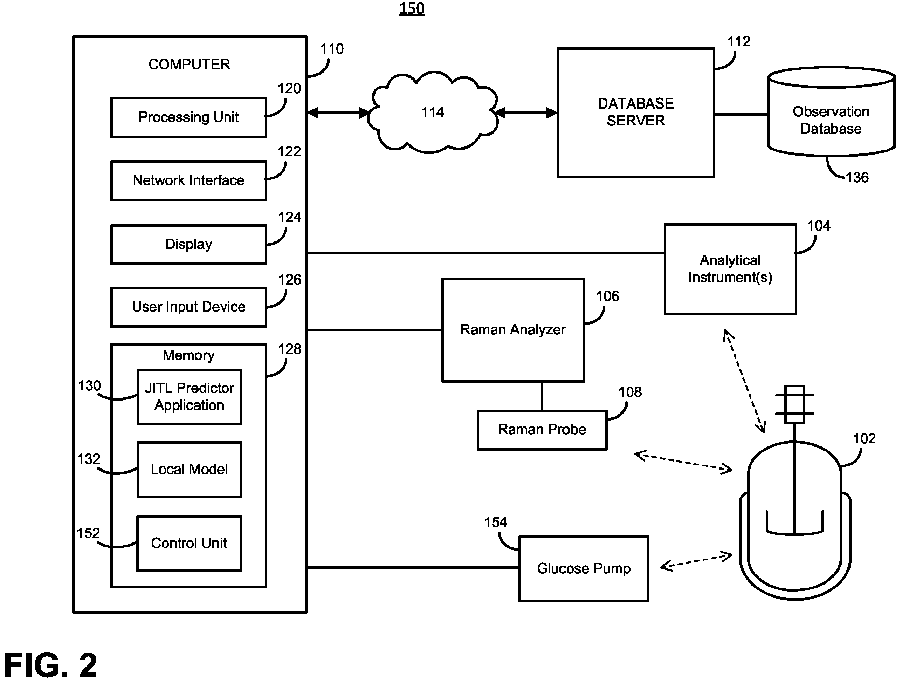

Some or all of the processes described above may be repeated a number of times over the life of the biopharmaceutical process in the bioreactor, in order to continuously monitor the process using a local model for which both calibration and maintenance are fully automated and in real-time. The analytical measurement(s) may be predicted for various purposes, depending on the embodiment and/or scenario. For example, certain parameters may be monitored (i.e., predicted) as a part of a quality control process, to ensure that the process still complies with relevant regulations. As another example, one or more parameters may be monitored/predicted to provide feedback in a closed-loop control system. For example, FIG. 2 depicts a system 150 that is similar to system 100, but attempts to control a glucose concentration in the biopharmaceutical process (i.e., attempts to make the predicted glucose concentration match a desired set point, within some acceptable tolerance). It is understood that, in other embodiments, system 150 may instead (or also) be used to control process parameters other than glucose level, or to control glucose level based on predictions of one or more other process parameters (e.g., lactate level). In FIG. 2, the same reference numbers are used to indicate the corresponding components from FIG. 1. For example, JITL predictor application 130 of FIG. 2 may be the same as JITL predictor application 130 of FIG. 1 (with the various units of JITL predictor application 130 not being shown in FIG. 2 for purposes of clarity).

As seen in FIG. 2, within system 150, memory 128 also stores a control unit 152. Control unit 152 is configured to control a glucose pump 154, i.e., to cause glucose pump 154 to selectively introduce additional glucose into the biopharmaceutical process within bioreactor 102. Control unit 152 may comprise software instructions that are executed by processing unit 120, for example, and/or appropriate firmware and/or hardware. In some embodiments, control unit 152 implements a model predictive control (MPC) technique, using glucose concentrations as inputs in a closed-loop architecture. As, according to the invention, local model 132 provides credibility bounds or other confidence indicators with each prediction (local model 132 is a Gaussian process model), control unit 152 may also accept the confidence indicators as inputs. For example, control unit 152 may only generate control instructions for glucose pump 154 based on glucose concentration predictions having a sufficiently high confidence indicator (e.g., only based on predictions associated with credibility bounds that do not exceed some percentage or absolute measurement range, or only based on predictions associated with confidence scores over some minimum threshold score, etc.), or may increase and/or reduce the weight of a given prediction based on its confidence indicator, etc.

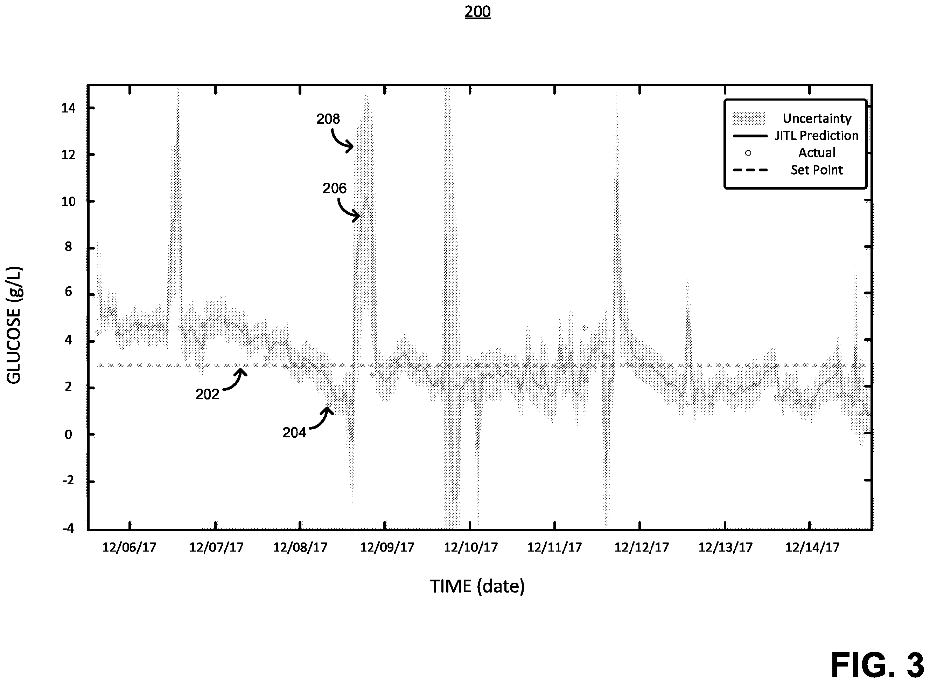

FIG. 3 depicts experimental results 200 for one example implementation in which JITL techniques were used to calibrate and maintain a local Gaussian process model. In the plot of FIG. 3, the horizontal, dashed line 202 represents the glucose concentration set point, the circles 204 represent actual measurements of glucose concentration (e.g., made by an analytical instrument similar to one of analytical instrument(s) 104 of FIG. 1), the solid line 206 represents the predicted measurements of glucose concentration (e.g., as predicted by a model similar to local model 132), and the shaded areas 208 represent credibility bounds (for 95% credibility) associated with the predicted measurements. As seen in FIG. 3, for a glucose concentration set point of 3 grams per liter (g/L), the predictions made using a JITL technique are generally in close agreement with the analytical measurements.

The process of conducting a query, and building/calibrating local model 132, will now be described mathematically in more detail, with reference to one specific JITL embodiment in which local model 132 is a Gaussian process model that uses a single Raman scan vector as an input and predicts a single analytical measurement:

Let $\mathcal{D} = \{b_j, \mathbf{a}_j\}_{j=1}^{J}$ (or $\mathcal{D} = \{\overline{\mathbf{b}}, \overline{\mathbf{a}}\}$ in compact notation) denote a set of ordered pairs of input and output data, such that $\overline{\mathbf{a}} \equiv \{\mathbf{a}_1, \mathbf{a}_2 \ldots, \mathbf{a}_J\}$ are the inputs and $\overline{\mathbf{b}} \equiv \{b_1, b_2 \ldots, b_J\}$ are the outputs. Further, it is assumed that $\mathbf{a}_j \in \mathbb{R}^{n_a}$ is an $n_a$-dimensional input vector, and $b_j \in \mathbb{R}$ is a scalar output. Physically, $\mathbf{a}_j \in \mathbb{R}^{n_a}$ can be thought of as a spectroscopic measurement (e.g., NIR or Raman), and $b_j \in \mathbb{R}$ as the analytical measurement for the state of interest (e.g., glucose or lactate concentration). Given a training data set $\mathcal{D}$, the objective of a spectroscopic model calibration problem is to identify the relationship between the inputs and outputs for the model of the form:

$$b_j = f(\mathbf{a}_j) + \boldsymbol{\epsilon}_j \qquad \text{Equation (1)}$$

where $f \in \mathbb{R}$ is the spectroscopic model, and $\varepsilon_j \sim \mathcal{N}(0, \sigma^2)$ is a zero-mean, normally-distributed measurement noise, with variance $\sigma^2$ being unknown. The standard practice in model calibration is to assume that $f(\cdot)$ is linear, and then use methods such as PLS to train the model. Instead of ascribing any limiting or fixed form to $f(\cdot)$, it is assumed here that $f(\cdot)$ is a latent function modeled as a Gaussian process, such that

$$f(\bar{\mathbf{a}}) \equiv \left( f(\mathbf{a}_1), f(\mathbf{a}_2), \dots, f(\mathbf{a}_J) \right) \sim \mathcal{GP}(\boldsymbol{\mu}_\theta(\bar{\mathbf{a}}), \mathbf{k}_\theta(\bar{\mathbf{a}}, \bar{\mathbf{a}}))$$

represents a random sample from a Gaussian process, with mean $\boldsymbol{\mu}_\theta(\bullet) \in \mathbb{R}^J$ and a covariance function $\mathbf{k}_\theta(\bullet, \bullet) \in \mathbb{R}^{J \times J}$, which are typically defined as follows:

$$\boldsymbol{\mu}_\theta(\bar{\mathbf{a}}) \equiv \left[ \mu_\theta(\mathbf{a}_1), \mu_\theta(\mathbf{a}_2) \dots, \mu_\theta(\mathbf{a}_J) \right]^T, \qquad \text{Equation (2a)}$$

$$k_\theta(\bar{a}; \bar{a}) \equiv \begin{bmatrix} k_\theta(a_1, a_1) & k_\theta(a_1, a_2) & \cdots & k_\theta(a_1, a_J) \\ k_\theta(a_2, a_1) & k_\theta(a_2, a_2) & \cdots & k_\theta(a_2, a_J) \\ \vdots & \vdots & \ddots & \vdots \\ k_\theta(a_J, a_1) & k_\theta(a_J, a_2) & \cdots & k_\theta(a_J, a_J) \end{bmatrix}. \qquad \text{Equation (2b)}$$

Also, $\theta \in \mathbb{R}^{n_\theta}$ denotes hyper-parameters for the Gaussian process model. A Gaussian process is a collection of random variables, any finite number of which have a joint Gaussian distribution, such that, for a set of finite inputs $\bar{\mathbf{a}} \equiv \{\mathbf{a}_1, \mathbf{a}_2, \dots, \mathbf{a}_J\}$ one can write:

$$p(\mathbf{f}|\bar{\mathbf{a}}) = \mathcal{N}\left( \mu_\theta(\bar{\mathbf{a}}), \mathbf{k}_\theta(\bar{\mathbf{a}}, \bar{\mathbf{a}}) \right) \qquad \text{Equation (3)}$$

The spectroscopic model calibration problem then reduces to learning the latent Gaussian process function $f \in \mathbb{R}$ using $\mathcal{D}$. For the sake of mathematical convenience, and general brevity, it is assumed here that $\mu_\theta = 0_{n_a}$; however, this need not be the case in general, and the results here can easily be extended to models with $\mu_\theta \neq 0_{n_a}$. The role of a covariance function in Gaussian processes is similar to that of the kernels used in support vector machines (SVM). A common choice for the covariance function is the Gaussian kernel, and is given by

$$k_\theta(\mathbf{a}_i, \mathbf{a}_j) = \beta \exp\left( -\frac{1}{2} \sum_{l=1}^{n_a} \frac{\left( a_i^{(l)} - a_j^{(l)} \right)^2}{\alpha_l} \right), \qquad \text{Equation (4)}$$

where $k_\theta(\mathbf{a}_i, \mathbf{a}_j) \in \mathbb{R}_+$ is the covariance between the input pair $\{\mathbf{a}_i, \mathbf{a}_j\}$. A Gaussian kernel $k_\theta(\mathbf{a}_i, \mathbf{a}_j)$ assigns a higher correlation if the inputs in the set $\{\mathbf{a}_i, \mathbf{a}_j\}$ are "close" to each other as defined by the Euclidean distance in Equation (4).
For the choice of a Gaussian kernel, Equation (4) is a positive definite symmetric matrix, such that $\mathbf{k}_\theta(\cdot, \cdot) \in \mathbb{S}_{++}^{J \times J}$. In Equation (4), the set $\theta \equiv \{\beta, \{\alpha_l\}_{l=1}^{n_a}\}$ is a set of hyperparameters. Physically, $\alpha_l \in \mathbb{R}_+$ is a length-scale parameter and $\beta \in \mathbb{R}_+$ is a signal-variance parameter. The choice of a Gaussian covariance function in Equation (4) corresponds to a prior assumption that $f$ is smooth and continuous. Thus, by varying the hyperparameters of the covariance function, the "smoothness" of f can be varied. Here, Gaussian processes with a Gaussian covariance function are assumed. However, this need not be the case in general.

Given $\mathcal{D}$, the objective is to learn the hyperparameters of the Gaussian process, including any other unknown model parameters. For the Gaussian process in Equation (1), the set of unknown parameters is $\gamma \equiv \{\theta, \sigma^2\} \in \Gamma \subseteq \mathbb{R}^{n_\gamma}$. The parameter-learning step may be performed by maximizing the marginalized likelihood (or evidence) function over the space of unknown parameters. For example, for the Gaussian process in Equation (1), a marginalized likelihood function is given as follows

$$p(\bar{\mathbf{b}}|\bar{\mathbf{a}}) = \int p(\bar{\mathbf{b}}|\mathbf{f}, \bar{\mathbf{a}})p(\bar{\mathbf{f}}|\bar{\mathbf{a}})d\mathbf{f}, \qquad \text{Equation (5)}$$

where $p(\bar{\mathbf{b}}|\bar{\mathbf{a}})$ is a marginalized likelihood function, $p(\bar{\mathbf{b}}|\bar{\mathbf{f}}, \bar{\mathbf{a}})$ is the likelihood function given by

$$p(\bar{\mathbf{b}}|\mathbf{f}, \bar{\mathbf{a}}) = \mathcal{N}\big(\mathbf{f}(\bar{\mathbf{a}}), \sigma^2 \mathbf{I}_{J \times J}\big), \qquad \text{Equation (6)}$$

and $p(\bar{\mathbf{f}}|\bar{\mathbf{a}})$ is the prior density function given in Equation (3). For a Gaussian likelihood and prior densities in Equations (6) and (3), respectively, the integral in Equation (5) has a closed-form solution, such that the marginalized likelihood function is given by

$$p(\bar{\mathbf{b}}|\bar{\mathbf{a}}) = \mathcal{N}\big(\mathbf{0}_J, \mathbf{k}_\theta(\bar{\mathbf{a}}, \bar{\mathbf{a}}) + \sigma^2 \mathbf{I}_{J \times J}\big). \qquad \text{Equation (7)}$$

Now given Equation (7), $\gamma \equiv \{\theta, \sigma^2\} \in \Gamma \subseteq \mathbb{R}^{n_\gamma}$ can be estimated by solving the following optimization problem:

$$\gamma^* \in \arg\max \log p(\bar{\mathbf{b}}|\bar{\mathbf{a}}), \qquad \text{Equation (8)}$$

where $\gamma^* \in \Gamma$ is an optimal estimate. From Equation (7), we have

$$\log p(\bar{\mathbf{b}}|\bar{\mathbf{a}}) = -\tfrac{1}{2}\mathbf{b}^{-\mathrm{T}}\mathbf{k}_\gamma^{-1}\bar{\mathbf{b}} - \tfrac{1}{2}\log|\mathbf{k}_\gamma| - \tfrac{J}{2}\log 2\pi, \qquad \text{Equation (9)}$$

where $\mathbf{k}_\gamma \equiv \mathbf{k}_\theta(\bar{\mathbf{a}}|\bar{\mathbf{a}}) + \sigma^2 \mathbf{I}_{J \times J}$. To solve the optimization problem in Equation (8), the partial derivatives of Equation (9) are determined with respect to $\gamma$ such that for all $r = 1, 2, ..., n_\gamma$,

$$\frac{\partial}{\partial \gamma_r}\log p(\bar{\mathbf{b}}|\bar{\mathbf{a}}) = \tfrac{1}{2}\mathbf{b}^{-\mathrm{T}}\mathbf{k}_\gamma^{-1}\frac{\partial \mathbf{k}_\gamma}{\partial \gamma_r}\mathbf{k}_\gamma^{-1}\bar{\mathbf{b}} - \tfrac{1}{2}\mathrm{Tr}\left[\mathbf{k}_\gamma^{-1}\frac{\partial \mathbf{k}_\gamma}{\partial \gamma_r}\right], \qquad \text{Equation (10a)}$$

$$= \tfrac{1}{2}\mathrm{Tr}\left(\big(\alpha\alpha^{\mathrm{T}} - \mathbf{k}_\gamma^{-1}\frac{\partial \mathbf{k}_\gamma}{\partial \gamma_r}\big)\right), \qquad \text{Equation (10b)}$$

where $\alpha = \mathbf{k}_\gamma^{-1}\bar{\mathbf{b}}$. Given a marginalized likelihood function in Equation (7) and its derivatives in Equation (10b), a gradient-descent method can be used to solve Equation (8). Because Equation (8) is generally a non-convex optimization problem with multiple local optima, caution must be exercised while solving the optimization problem. It is assumed here that $\gamma^*$ is known or can be computed by solving Equation (8). Further, to ease the notational burden, it will be assumed here that $\gamma$ is the optimal estimate $\gamma^*$, unless specified otherwise.

Once the Gaussian process spectroscopic calibration model in Equation (1) is trained, it can be deployed for real-time predictive applications. As before, let $\mathcal{D}$ be the training data set used to train the Gaussian process model, and let $\mathbf{a}^* \in \mathbb{R}^{n_a}$ be a new test spectroscopic signal. The objective is then to predict an output $b^* \in \mathbb{R}$ corresponding to the test input $\mathbf{a}^*$. The first step in computing $b^*$ is to construct a joint density of all the training output set $\bar{\mathbf{b}}$ and the test Gaussian process output $f(\mathbf{a}^*)$ conditioned on the training input set $\bar{\mathbf{a}}$ and the test input $\mathbf{a}^*$. This joint density is given as follows:

$$p\big(\bar{\mathbf{b}}\big|f(\mathbf{a}^*)\big)|\bar{\mathbf{a}}, \mathbf{a}^*) = \mathcal{N}\left(0, \begin{bmatrix} \mathbf{k}_\gamma(\bar{\mathbf{a}}, \bar{\mathbf{a}}) & \mathbf{k}_\theta(\bar{\mathbf{a}}, \mathbf{a}^*) \\ \mathbf{k}_\theta(\mathbf{a}^*, \bar{\mathbf{a}}) & k_\theta(\mathbf{a}^*, \mathbf{a}^*) \end{bmatrix}\right), \qquad \text{Equation (11)}$$

where $\mathbf{k}_\gamma \equiv \mathbf{k}_\theta(\bar{\mathbf{a}}, \bar{\mathbf{a}}) + \sigma^2 \mathbf{I}_{J \times J}$. Given Equation (11), under the Bayesian framework, the Gaussian process output $f(\mathbf{a}^*)$ is calculated by constructing a distribution over all Gaussian process outputs. In other words, we seek a posterior distribution for the Gaussian process output $f(\mathbf{a}^*)$. Of course, the posterior distribution over $f(\mathbf{a}^*)$ need only include those functions which agree with the training set $\mathcal{D}$. Under probabilistic settings, a posterior distribution over $f(\mathbf{a}^*)$ can be computed by conditioning the joint distribution in Equation (11) on the training set $\mathcal{D}$ to give

$$p(f(\mathbf{a}^*)|\mathcal{D}, \mathbf{a}^*) = \mathcal{N}\left(\mu_\theta^*, k_\theta^*\right), \qquad \text{Equation (12)}$$

where $p(f(\mathbf{a}^*)|\mathcal{D}, \mathbf{a}^*)$ is a posterior distribution for the Gaussian process output, and $\mu_\theta^* \equiv \mathbb{E}\left[\left(f(\mathbf{a}^*)\mid D, \mathbf{a}^*\right)\right]$ is given by

$$\mu_\theta^* = \mathbf{k}_\theta(\mathbf{a}^*, \bar{\mathbf{a}})\left[\mathbf{k}_\gamma(\bar{\mathbf{a}}, \bar{\mathbf{a}})\right]^{-1}\bar{\mathbf{b}}, \qquad \text{Equation (13)}$$

and $k_\theta^* \equiv \mathbb{V}\left[(f(\mathbf{a}^*)|\mathcal{D}, \mathbf{a}^*)\right]$ is given by

$$k_\theta^* = k_\theta(\mathbf{a}^*, \mathbf{a}^*) - \mathbf{k}_\theta(\mathbf{a}^*, \bar{\mathbf{a}})\left[\mathbf{k}_\gamma(\bar{\mathbf{a}}, \bar{\mathbf{a}})\right]^{-1}\mathbf{k}_\theta(\bar{\mathbf{a}}, \mathbf{a}^*). \qquad \text{Equation (14)}$$

Given Equation (12), a predictive posterior distribution for the output $b^*$ can be computed as follows

$$p(b^*|\mathcal{D}, \mathbf{a}^*) = \mathcal{N}\left(\mu_\theta^*, k_\theta^* + \sigma^2\right), \qquad \text{Equation (15)}$$

where $\mu_\theta^*$ and $k_\theta^*$ are given in Equations (13) and (14), respectively. For a single test input $\mathbf{a}^* \in \mathbb{R}^{n_\alpha}$ the Gaussian process prediction in Equation (15) gives a distribution of outputs that have a non-zero probability of being realized. In real-time applications, such as control and monitoring, one is likely interested in a point-estimate rather than the entire distribution. A point-estimate can be computed using a decision-theoretic approach. It can be shown that for a Gaussian posterior distribution in Equation (15), the mean function minimizes both the expected absolute and the square risk functions, with $\hat{b} = \mu_\theta^*$ being the most probable output for the input $\mathbf{a}^*$. Further, for the choice of $\hat{b} = \mu_\theta^*$ as the prediction, an approximately 95% credibility interval is given by

$$b^L = \left(\mu_\theta^* - 2\left(\sqrt{k_\theta^* + \sigma^2}\right)\right) \leq \hat{b} \leq \mu_\theta^* + 2\left(\sqrt{k_\theta^* + \sigma^2}\right) = b^U. \qquad \text{Equation (16)}$$

The interval in Equation (16) can be used to assess the quality of Gaussian process predictions, and/or in designing Gaussian process-based model predictive control or other robust monitoring strategies.

Turning now to the selection of relevant samples (here, observation data sets) in response to a query, the problem is, for a given query point $\mathbf{a}^* \in \mathbb{R}^{n_\alpha}$, and a central database/library $\mathcal{L} \equiv \{b_i, \mathbf{a}_i\}_{i=1}^{L_t}$ containing $L \in \mathbb{N}$ input-output pairs (observation data sets), to select a local training set $\mathcal{D} \equiv \{b_j, \mathbf{a}_j\}_{j=1}^{D}$ at time $t \in \mathbb{N}$ containing $D \in \mathbb{N}$ samples, where $D << L$. It is assumed that $\mathcal{L}$ is dynamic, and may include different entries during a campaign. There are numerous ways to construct $\mathcal{D}$ from $\mathcal{L}$. For purposes of this analysis, $\mathcal{D}$ is selected based on Euclidean distance between the spectra (e.g., Raman scan vectors) in set $\mathcal{L}$. While Euclidean-based similarity measures in a JITL framework have been reported to be sub-optimal in certain situations, they may be a beneficial choice when a Gaussian process model is used. This is because the Gaussian process model is itself based on Euclidean distance. The Gaussian kernel assigns a higher correlation only if the inputs in the set $\{\mathbf{a}_i, \mathbf{a}_j\}$ are "close" to each other. Therefore, by creating a local training set D with all the inputs being "close" to the query point, one can ensure that the local Gaussian process model captures the maximum "correlation" to predict the output at the query point.

An example algorithm that formally outlines the method to create a local training set $\mathcal{D}$ from $\mathcal{L}$, train the Gaussian process model using that training set, and make a prediction using the trained model is provided below in Algorithm 1:

1.  **Input:** Library $\mathcal{L} = \{(\mathbf{a}_i, \mathbf{b}_i)\}_{i=1}^{L}$, query point $\mathbf{a}^*$

2.  **Output:** Prediction $\hat{b}$ and uncertainty $(b^L, b^U)$

3.  **for** $t = 1$ to $T$ **do**

4.      Set $I \leftarrow \text{sample\_index}(\mathcal{L})$ and $\mathcal{D} \leftarrow \{\emptyset\}$

5.      **for** $d = 1$ to $D$ **do**

6.          $k_* \in \arg\max_{i \in I} \exp(-\|\mathbf{a}_i - \mathbf{a}^*\|)$

7.          $\mathcal{D} \leftarrow \mathcal{D} \cup \{\mathbf{a}_{k_*}, b_{k_*}\}$

8.          $I \leftarrow I \backslash \{i\}$

9.      **end for**

10.     Train Gaussian process model of Equation (1) using $\mathcal{D}$ and estimate $\gamma^*$

11.     Compute $\hat{b}$ and $(b^L, b^U)$ using Equations (13) and (16)

12. **end for**

**Algorithm 1**

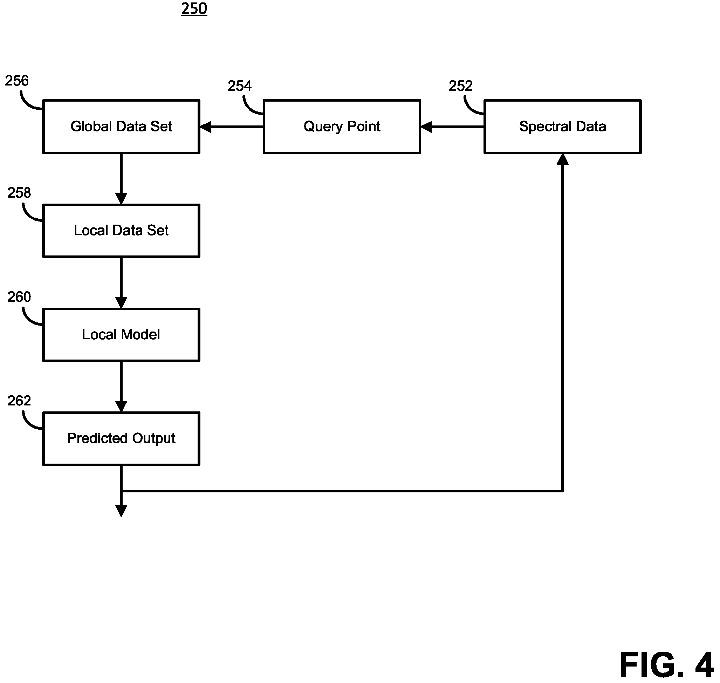

Turning now to FIG. 4, an example data flow 250 that may occur when analyzing a biopharmaceutical process using a JITL technique as described herein is shown. The data flow 250 may occur within system 100 of FIG. 1 or system 150 of FIG. 2, for example. In the data flow 250, spectral data 252 is provided by a spectrometer/probe. For example, spectral data 252 may include a Raman scan vector generated by Raman analyzer 106, or an NIR scan vector, etc. A query point 254 is generated (e.g., by query unit 140) based on spectral data 252, and is used to query a global data set 256, which may include all of the observation data sets in observation database 136, for example. Based on the query, a local data set 258 is identified within global data set 256. Local data set 258 may be selected based on relevancy criteria (e.g., Euclidean distance), for example, as described above.

Local data set 258 is then used as training data (e.g., by local model generator 142) to calibrate a local model 260 (e.g., local model 132). Local model 132 is then used (e.g., by prediction unit 144) to predict an output (analytical measurement) 262, such as a media component concentration, media state (e.g., glucose, lactate, glutamate, glutamine, ammonia, amino acids, Na+, K+ and other nutrients or metabolites, pH, $pCO_2$, $pO_2$, temperature, osmolality, etc.), viable cell density, titer, critical quality attributes, cell state, etc., and possibly also output credibility bounds or another suitable confidence indicator.

While a JITL-based local model (e.g., as in Algorithm 1 and data flow 250) provides a robust, nonlinear modeling framework, such an approach does not have an inherent mechanism for adaptation to time-varying process changes. To address this shortcoming, some embodiments may use an "adaptive" JITL (A-JITL) strategy. As noted above, new samples may be included in $\mathcal{L}$ as those samples become available. In such embodiments (i.e., where $\mathcal{L}$ is dynamic), $\mathcal{L}$ may be denoted as $\mathcal{L}_t$. In one such embodiment, a moving time-window method is implemented, in which a newly obtained sample is added to $\mathcal{L}_t$ and the oldest sample is removed from $\mathcal{L}_t$. Discarding the oldest sample may be beneficial because, in adaptive strategies, maintaining the size of $\mathcal{L}_t$ can be critical to ensure computational tractability of the overall JITL framework. One major concern with this approach, however, is that simply discarding old samples can lead to information loss, as old samples may contain relevant information.

To avoid such information loss, in one embodiment, new samples are added to $\mathcal{L}_t$ without removing any old/existing samples. Thus, the central database $\mathcal{L}_t$ expands with an increasing number of samples as new analytical measurements become available. In a cell culture process application, an expanding database may not give rise to any significant computational issues, due to the fact that such processes are typically operated as batch processes with two to three weeks of batch-time. This naturally limits the number of new samples that are to be included in $\mathcal{L}_t$. Further, only a limited number of analytical measurements are typically sampled during the course of a cell culture process batch (unlike, for instance, chemical industries in which analytical measurements are frequently sampled). Thus, there would typically only be a modest increase in the size of the database $Lt$, without any significant bearing on the computational stability of the overall JITL framework.

While including new samples in $\mathcal{L}_t$ is important for the continuous adaptation of Algorithm 1 (above), the success of this approach relies on the selection of those new samples in local database $\mathcal{D}$ for local model calibration. Algorithm 1, which selects samples for $\mathcal{D}$ from $\mathcal{L}$ based on Euclidean distance (e.g., line 6 of Algorithm 1), can be referred to as a "relevant-in-space" approach, as it only prioritizes samples that are relevant (close) in space. If new samples are not close to the query sample, as is likely the case when an abrupt set-point change (or other abrupt process condition change) occurs, Algorithm 1 may fail to include those samples in $\mathcal{D}$. Recursive methods (e.g., regularized partial least squares (RPLS), recursive least squares (RLS), and recursive N-way partial least squares (RNPLS)), on the other hand, are "relevant-in-time" because they prioritize the latest measurements, irrespective of relevance in space. Updating the local model using the latest samples can allow recursive methods to successfully adapt to current process conditions.

One such embodiment, referred to herein as "adaptive" JITL (A-JITL), prioritizes samples that are relevant both in space and time. Letting $\mathcal{G}^- = \{\{\mathbf{a}_i^-, \mathbf{b}_i^-\}\}_{i=1}^L$ represent a set of $L$ historical measurements available from before the start of a current experiment (i.e., the experiment/process in which query $\mathbf{a}^*$ occurs), and letting $\mathcal{G}^+ = \{\{\mathbf{a}_j^+, \mathbf{b}_j^+\}\}_{j=1}^n$ represent a set of n measurements available from the current experiment, the samples may be redistributed as follows:

$$\mathcal{L}_t = \mathcal{G}^- \cup \mathcal{G}^+ \setminus \{\{\mathbf{a}_j^+, \mathbf{b}_j^+\}\}_{j=n-k+1}^n , \qquad \text{Equation (17a)}$$

$$\mathcal{K} = \{\{\mathbf{a}_j^+, \mathbf{b}_j^+\}\}_{j=n-k+1}^n , \qquad \text{Equation (17b)}$$

where $\mathcal{L}_t$ represents the central database and $\mathcal{K}$ represents a set of the last (most recent) $k$ measurements. In some embodiments, $\mathcal{K}$ contains the last $k$ samples from the current experiment/process, and $\mathcal{L}_t$ contains samples from previous experiments/processes, as well as (potentially) samples from the current experiment/process that are older than the last $k$ samples. Equations (17a) and (17b) above are defined for a given query $\mathbf{a}^*$. For a query arriving at another time instant, datasets $\mathcal{L}_t$ and $\mathcal{K}$ may contain different samples, depending on the number of measurements available at that time instant. For example, once the sample ( $\mathbf{a}_{n+1}^+, \mathbf{b}_{n+1}^+$ ) is available, ( $\mathbf{a}_{n-k+1}^+, \mathbf{b}_{n-k+1}^+$ ) is removed from $\mathcal{K}$ and ( $\mathbf{a}_{n+1}^+, \mathbf{b}_{n+1}^+$ ) is included in $\mathcal{K}$. The discarded sample ( $\mathbf{a}_{n-k+1}^+, \mathbf{b}_{n-k+1}^+$ ) is then included in $\mathcal{L}_t$ to prevent any information loss. Updating $\mathcal{K}$ with the latest measurements ensures that $\mathcal{K}$ reflects at least some current conditions.

Given $\mathcal{L}_t$ and $\mathcal{K}$, the objective is to select $\mathcal{D}$. As noted above, for A-JITL, both space- and time-relevant samples are included in $\mathcal{D}$. If it is assumed that $\mathcal{D}$ can be decomposed as:

$$\mathcal{D} \equiv \mathcal{D}_S \cup \mathcal{D}_T, \qquad \text{Equation (18)}$$

where $\mathcal{D}_S$ and $\mathcal{D}_T$ are the space- and time-relevant sets, respectively, then the goal is to select $\mathcal{D}_S$ and $\mathcal{D}_T$. First, it is assumed that $\mathcal{D}_S \cap \mathcal{D}_T = 0$, such that $\mathcal{D}$ only contains unique samples. To design $\mathcal{D}_S$, $D$ - $k$ samples are selected from $\mathcal{L}_t$ based on a distance-based (spatial) metric, such as a "similarity index" or "s-value":

$$s_i = \text{sim}(\mathbf{a}_i, \mathbf{a}^*) = \exp(-\|\mathbf{a}_i - \mathbf{a}^*\|). \qquad \text{Equation (19)}$$

Equation (19) may be used as the similarity metric in the (non-adaptive) JITL technique described above, for example. Thus, for example, the $D$ - $k$ samples with the largest s-values may be selected from $\mathcal{L}_t$ for inclusion in $\mathcal{D}_S$. To design $\mathcal{D}_T$, if it is assumed that the last k samples from the current experiment/process are relevant in time, $\mathcal{D}_T$ may in some embodiments be defined as being equal to $\mathcal{K}$. It is noted that, unlike s-values that determine the membership of samples in $\mathcal{D}_S$, membership in $\mathcal{D}_T$ is decided based on sampling times. Of course, depending on the scenario, samples in $\mathcal{D}_T$ may exhibit large s-values. Irrespective of the s-values, $\mathcal{D}_T$ is only assumed to be relevant in time. Similarly, $\mathcal{D}_S$ is only relevant in space, because by construction, $\mathcal{L}_t$ has no time relevance. It is noted that $\mathcal{D}_S$ and $\mathcal{D}_T$ are defined for a given query $\mathbf{a}^*$, samples in $\mathcal{D}_S$ are selected based on their s-values computed with respect to $\mathbf{a}^*$, and samples in $\mathcal{D}_T$ are selected based on their sampling times computed relative to the sampling time of $\mathbf{a}^*$. For convenience, $\mathcal{D}_S$ and $\mathcal{D}_T$ are generically defined as follows:

$$\mathcal{D}_S \equiv \{\bar{\mathbf{a}}_S, \bar{\mathbf{b}}_S\}, \qquad \text{Equation (20a)}$$

$$\mathcal{D}_T \equiv \{\bar{\mathbf{a}}_T, \bar{\mathbf{b}}_T\}, \qquad \text{Equation (20b)}$$

where $\bar{\mathbf{a}}_S$ and $\bar{\mathbf{a}}_T$ are the space- and time-relevant samples from the Raman spectrometer, respectively, and $\bar{\mathbf{b}}_S$ and $\bar{\mathbf{b}}_T$ are the space- and time-relevant samples from the analytical instrument, respectively, such that

$$\bar{\mathbf{a}}_S \equiv [\mathbf{a}_1, \dots, \mathbf{a}_{D-k}]^{\mathrm{T}}; \ \bar{\mathbf{a}}_T \equiv [\mathbf{a}_{D-k+1}, \dots, \mathbf{a}_D]^{\mathrm{T}}, \qquad \text{Equation (21a)}$$

$$\bar{\mathbf{b}}_S \equiv [b_1, \dots, b_{D-k}]^{\mathrm{T}}; \ \bar{\mathbf{b}}_T \equiv [b_{D-k+1}, \dots, b_D]^{\mathrm{T}}. \qquad \text{Equation (21b)}$$

Substituting Equations (20a) and (20b) into Equation (18) gives set $\mathcal{D}$, denoted generically as $\mathcal{D} \equiv \{\bar{\mathbf{a}}, \bar{\mathbf{b}}\}$, where $\bar{\mathbf{a}} \equiv [\bar{\mathbf{a}}_S, \bar{\mathbf{a}}_T]^{\mathrm{T}}$ and $\bar{\mathbf{b}} \equiv [\bar{\mathbf{b}}_S, \bar{\mathbf{b}}_T]^{\mathrm{T}}$. In contrast to the (non-adaptive) JITL technique discussed above, the local library/dataset D prioritizes samples that are relevant in space and time. Given $\mathcal{D}_T$ and a query $\mathbf{a}^*$, the Gaussian process model in Equation (1) (e.g., local model 132) can be calibrated. The point estimate and the credibility interval at $\mathbf{a}^*$ can be computed using Equations (13) and (16), respectively, where $\mathbf{k}_\gamma(\bar{\mathbf{a}}, \bar{\mathbf{a}})$ and $\mathbf{k}_\theta(\mathbf{a}^*, \bar{\mathbf{a}})$ are given by

$$\mathbf{k}_\gamma(\bar{\mathbf{a}}, \bar{\mathbf{a}}) \equiv \begin{bmatrix} \mathbf{k}_\theta(\bar{\mathbf{a}}_S, \bar{\mathbf{a}}_S) & \mathbf{k}_\theta(\bar{\mathbf{a}}_S, \bar{\mathbf{a}}_T) \\ \mathbf{k}_\theta(\bar{\mathbf{a}}_T, \bar{\mathbf{a}}_S) & \mathbf{k}_\theta(\bar{\mathbf{a}}_T, \bar{\mathbf{a}}_T) \end{bmatrix} + \sigma^2 \mathbf{I}_{D \times D}, \qquad \text{Equation (22a)}$$

$$\mathbf{k}_\theta(\mathbf{a}^*, \bar{\mathbf{a}}) \equiv [\mathbf{k}_\theta(\mathbf{a}^*, \bar{\mathbf{a}}_S) \quad \mathbf{k}_\theta(\mathbf{a}^*, \bar{\mathbf{a}}_T)], \qquad \text{Equation (22b)}$$

where $\mathbf{k}_\theta(\bar{\mathbf{a}}_S, \bar{\mathbf{a}}_S) \in S_+^{(D-k)}$ and $\mathbf{k}_\theta(\bar{\mathbf{a}}_T, \bar{\mathbf{a}}_T) \in S_+^k$ are the covariance functions associated with $\mathcal{D}_S$ and $\mathcal{D}_T$, respectively, and where $\mathbf{k}_\theta(\bar{\mathbf{a}}_S, \bar{\mathbf{a}}_T) \in \mathbb{R}^{(D-k)k}$ is covariance between $\mathcal{D}_S$ and $\mathcal{D}_T$.

An example algorithm that formally outlines the A-JITL technique is provided below in Algorithm 2:

1.      **Input:** Library $\mathcal{L}_t = \{(\mathbf{a}_i, \mathrm{b}_i)\}_{i=1}^{L}$, query point $\mathbf{a}^*$

2.      **Output:** Prediction $\hat{b}$ and uncertainty $(b^L, b^U)$

3.      Set $\mathcal{K} \leftarrow \{\emptyset\}$

4.      **for** $t = 1$ to $T$ **do**

5.              Set $I \leftarrow \mathrm{sample\_index}(\mathcal{L}_t)$, $\mathcal{D}_S \leftarrow \{\emptyset\}$, $\mathcal{D}_T \leftarrow \{\emptyset\}$

6.              **for** $d = 1$ to $D - \mathrm{set\_cardinality}(\mathcal{K})$ **do**

7.                      $i_* \in \arg\max_{i \in I} \mathrm{sim}(\mathbf{a}_i, \mathbf{a}^*)$

8.                      $\mathcal{D}_S \leftarrow \mathcal{D}_S \cup \{\mathbf{a}_{i_*}, \mathrm{b}_{i_*}\}$

9.                      $I \leftarrow I \setminus \{i_*\}$

10.             **end for**

11.             **if** $\mathrm{set\_cardinality}(\mathcal{K}) \geq 1$ **then**

12.                     $\mathcal{D}_T \leftarrow \mathcal{K}$

13.             **end if**

14.             $\mathcal{D} \leftarrow \mathcal{D}_S \cup \mathcal{D}_T$

15.             Train Gaussian process model in Equation (1) using $\mathcal{D}$ and estimate $\gamma^*$

16.             Compute $\hat{b}$ and $(b^L, b^U)$ using Equations (13) and (16)

17.             **if** $b^*$ is available **then**

18.                     **if** $\mathrm{size}(\mathcal{K}) = k$ **then**

19.                             $\mathcal{L}_t \leftarrow \mathcal{L}_t \cup \mathrm{select\_oldest}(\mathcal{K})$

20.                             $\mathcal{K} \leftarrow \mathrm{delete\_oldest}(\mathcal{K})$

21.                             $\mathcal{K} \leftarrow \mathcal{K} \cup \{\mathbf{a}^*, b^*\}$

22.                     **end if**

23.                     $\mathcal{K} \leftarrow \mathcal{K} \cup \{\mathbf{a}^*, b^*\}$

24.             **end if**

25.     **end for**

**Algorithm 2**

Thus, Algorithm 2 combines JITL (relevant-in-space) with recursive learning (relevant-in-time). For $|\mathcal{D}_T| = 0$, for example, calibration of local model 132 using Algorithm 2 is similar to relevant-in-space JITL, whereas for $|\mathcal{D}_S| = 0$, calibration of local model 132 using Algorithm 2 is similar to recursive learning. Thus, by adjusting $|\mathcal{D}_S|$ and $|\mathcal{D}_T|$, the (non-recursive) JITL and recursive learning can be appropriately balanced.

Turning now to FIG. 5, an example data flow 300 that may occur when analyzing a biopharmaceutical process using an A-JITL technique as described herein is shown. The data flow 300 may occur within system 100 of FIG. 1 or system 150 of FIG. 2, for example. In the data flow 300, spectral data 302 is provided by a spectrometer/probe. For example, spectral data 302 may include a Raman scan vector generated by Raman analyzer 106, or an NIR scan vector, etc. A query point 304 is generated (e.g., by query unit 140) based on spectral data 302, and is used to query a global data set 306, which may include all of the observation data sets in observation database 136, for example. Global data set 306 is logically separated into the last k entries 307A (e.g., all from the current experiment/process), and all entries 307B prior to the last k entries 307A (e.g., from previous experiments/processes, and possibly also the current experiment/process). The value of $k$ may be determined based on the sample number of the query point 304. As used herein, the term "sample number" may broadly refer to any indicator of the time, or the relative time, associated with a given sample/observation. Certain entries among entries 307B are added to local data set 308 based on spatial similarity (e.g., Euclidean distance) to the query point 304, while all entries 307A may be added to local data set 308 irrespective of spatial similarity. Local data set 308 may be generated from entries 307A and entries 307B in accordance with Algorithm 2, for example.

Local data set 308 is then used as training data (e.g., by local model generator 142) to calibrate a local model 310 (e.g., local model 132). Local model 310 is then used (e.g., by prediction unit 144) to predict an output (analytical measurement) 312, such as a media component concentration, media state (e.g., glucose, lactate, glutamate, glutamine, ammonia, amino acids, Na+, K+ and other nutrients or metabolites, pH, pCO2, pO2, temperature, osmolality, etc.), viable cell density, titer, critical quality attributes, cell state, etc., and possibly also output credibility bounds or another suitable confidence indicator.

If an actual analytical measurement (e.g., a measurement made by an analytical instrument such as one of analytical instrument(s) 104) is available, a new entry 314 is created and added to global data set 306. Such measurements may be available on a periodic sampling basis (e.g., once or twice per day), for example, and/or may be made available in response to a trigger with variable timing (e.g., if a certain number of predictions in a row have unacceptably wide credibility bounds, etc.), as discussed further below.

While including space- and time-relevant samples in $\mathcal{D}$ is necessary for the continuous adaptation of the A-JITL approach discussed above, the overall degree of adaptation achieved by A-JITL depends on how effectively $\mathcal{D}$ is utilized for local model calibration. For a query sample/point, $\mathbf{a}^*$, a space-relevant sample $(\mathbf{a}_i, b_i) \in \mathcal{D}_S$ provides high correlation between the functions $(f(\mathbf{a}^*), f(\mathbf{a}_i))$. This is because, for a query $\mathbf{a}^*$, the space-relevance of $(\mathbf{a}_i, b_i)$ and the correlation between $(f(\mathbf{a}^*), f(\mathbf{a}_i))$ are both computed based on the Euclidean distance between $(\mathbf{a}_i, \mathbf{a}^*)$. Thus, for the choice of Euclidean-based similarity measure in Equation (19), and a Euclidean-based kernel in Equation (4), samples in $\mathcal{D}_S$ are expected to provide high functional correlations. Conversely, a time-relevant sample, $(\mathbf{a}_j, b_j) \in \mathcal{D}_T$ may not provide strong correlation between the functions $(f(\mathbf{a}^*), f(\mathbf{a}_j))$. This is because, as noted above, samples in $\mathcal{D}_T$ are not necessarily relevant in space. As a result, the correlation ascribed by the Gaussian kernel in Equation (4) between $(f(\mathbf{a}^*), f(\mathbf{a}_j))$ will be small if the space-relevance of $(\mathbf{a}_j, b_j)$ is small. From a modeling perspective, training a Gaussian process model in Equation (1) with samples bearing small correlations is undesirable, as this leads to poor model performance. Mathematically, this can be demonstrated as follows.

For a query $\mathbf{a}^*$ and a calibrated Gaussian process model of Algorithm 2, the model prediction $\hat{b}$ can be computed using Equation (13). Without loss of generality, if $\sigma^2 = 0$ (the noise-free case), one can write Equation (13) as follows:

$$\hat{b} = \begin{bmatrix} \mathbf{k}_\theta(\bar{\mathbf{a}}_S, \mathbf{a}^*) \\ \mathbf{k}_\theta(\bar{\mathbf{a}}_T, \mathbf{a}^*) \end{bmatrix}^T \begin{bmatrix} \mathbf{k}_\theta(\bar{\mathbf{a}}_S, \bar{\mathbf{a}}_S) & \mathbf{k}_\theta(\bar{\mathbf{a}}_S, \bar{\mathbf{a}}_T) \\ \mathbf{k}_\theta(\bar{\mathbf{a}}_T, \bar{\mathbf{a}}_S) & \mathbf{k}_\theta(\bar{\mathbf{a}}_T, \bar{\mathbf{a}}_T) \end{bmatrix}^{-1} \begin{bmatrix} \bar{\mathbf{b}}_S \\ \bar{\mathbf{b}}_T \end{bmatrix}. \qquad \text{Equation (23)}$$

If $(\bar{\mathbf{a}}_T, \bar{\mathbf{b}}_T)$ has negligible space relevance (i.e., the s-value between $\bar{\mathbf{a}}_T$ and $\mathbf{a}^*$ is infinitely large, then Equation 4 results in $\mathbf{k}_\theta(\mathbf{a}^*, \bar{\mathbf{a}}_T) \approx 0_{1xk}$. Further, by construction, since $\bar{\mathbf{a}}_S$ is closer to $\mathbf{a}^*$ than to $\bar{\mathbf{a}}_T$, the result is $\mathbf{k}_\theta(\mathbf{a}_S, \bar{\mathbf{a}}_T) \approx 0_{(D-k)xk}$ and $\mathbf{k}_\theta(\bar{\mathbf{a}}_T, \bar{\mathbf{a}}_S) \approx 0_{kx(D-k)}$. Substituting these into Equation (23) yields

$$\hat{b} \approx \begin{bmatrix} \mathbf{k}_\theta(\bar{\mathbf{a}}_S, \mathbf{a}^*) \\ 0_{kx1} \end{bmatrix}^T \begin{bmatrix} \mathbf{k}_\theta(\bar{\mathbf{a}}_S, \bar{\mathbf{a}}_S) & 0_{(D-k)xk} \\ 0_{kx(D-k)} & \mathbf{k}_\theta(\bar{\mathbf{a}}_T, \bar{\mathbf{a}}_T) \end{bmatrix}^{-1} \begin{bmatrix} \bar{\mathbf{b}}_S \\ \bar{\mathbf{b}}_T \end{bmatrix}, \qquad \text{Equation (24a)}$$

$$= \begin{bmatrix} \mathbf{k}_\theta(\bar{\mathbf{a}}_S, \mathbf{a}^*) \\ 0_{kx1} \end{bmatrix}^T \begin{bmatrix} \mathbf{k}_\theta^{-1}(\bar{\mathbf{a}}_S, \bar{\mathbf{a}}_S) & 0_{(D-k)xk} \\ 0_{kx(D-k)} & \mathbf{k}_\theta^{-1}(\bar{\mathbf{a}}_T, \bar{\mathbf{a}}_T) \end{bmatrix} \begin{bmatrix} \bar{\mathbf{b}}_S \\ \bar{\mathbf{b}}_T \end{bmatrix} \qquad \text{Equation (24b)}$$

$$= \mathbf{k}_\theta(\mathbf{a}^*, \bar{\mathbf{a}}_S) \mathbf{k}_\theta^{-1}(\bar{\mathbf{a}}_S, \bar{\mathbf{a}}_S) \bar{\mathbf{b}}_S. \qquad \text{Equation (24c)}$$

From Equation (24c) it is clear that the point estimate is independent of $\mathcal{D}_T$. Similarly, it can be shown that Equation (16) is also independent of $\mathcal{D}_T$. For example, $k_\theta^*$ in Equation (16) can be computed as follows:

$$-k_\theta^* + k_\theta(\mathbf{a}^*, \mathbf{a}^*) = \mathbf{k}_\theta(\mathbf{a}^*, \bar{\mathbf{a}}) [\mathbf{k}_\gamma(\bar{\mathbf{a}}, \bar{\mathbf{a}})]^{-1} \mathbf{k}_\theta(\bar{\mathbf{a}}, \mathbf{a}^*), \qquad \text{Equation (25a)}$$

$$\approx \begin{bmatrix} \mathbf{k}_\theta(\bar{\mathbf{a}}_S, \mathbf{a}^*) \\ \mathbf{k}_\theta(\bar{\mathbf{a}}_T, \mathbf{a}^*) \end{bmatrix}^T \begin{bmatrix} \mathbf{k}_\theta(\bar{\mathbf{a}}_S, \bar{\mathbf{a}}_S) & \mathbf{k}_\theta(\bar{\mathbf{a}}_S, \bar{\mathbf{a}}_T) \\ \mathbf{k}_\theta(\bar{\mathbf{a}}_T, \bar{\mathbf{a}}_S) & \mathbf{k}_\theta(\bar{\mathbf{a}}_T, \bar{\mathbf{a}}_T) \end{bmatrix}^{-1} \begin{bmatrix} \mathbf{k}_\theta(\bar{\mathbf{a}}_S, \mathbf{a}^*) \\ \mathbf{k}_\theta(\bar{\mathbf{a}}_T, \mathbf{a}^*) \end{bmatrix}, \qquad \text{Equation (25b)}$$

$$= \begin{bmatrix} \mathbf{k}_\theta(\bar{\mathbf{a}}_S, \mathbf{a}^*) \\ 0_{kx1} \end{bmatrix}^T \begin{bmatrix} \mathbf{k}_\theta^{-1}(\bar{\mathbf{a}}_S, \bar{\mathbf{a}}_S) & 0_{(D-k)xk} \\ 0_{kx(D-k)} & \mathbf{k}_\theta^{-1}(\bar{\mathbf{a}}_T, \bar{\mathbf{a}}_T) \end{bmatrix} \begin{bmatrix} \mathbf{k}_\theta(\bar{\mathbf{a}}_S, \mathbf{a}^*) \\ 0_{kx1} \end{bmatrix}, \qquad \text{Equation (25c)}$$

$$k_\theta^* \approx k_\theta(\mathbf{a}^*, \mathbf{a}^*) - \mathbf{k}_\theta(\mathbf{a}^*, \bar{\mathbf{a}}_S) \mathbf{k}_\theta^{-1}(\bar{\mathbf{a}}_S, \bar{\mathbf{a}}_S) \mathbf{k}_\theta(\bar{\mathbf{a}}_S, \mathbf{a}^*). \qquad \text{Equation (25d)}$$

From Equations (25b) and (25c) it can be seen that several approximations are used, including $\mathbf{k}_\theta(\mathbf{a}^*, \overline{\mathbf{a}}_T) \approx 0_{k\times 1}$, $\mathbf{k}_\theta(\mathbf{a}_S, \overline{\mathbf{a}}_T) \approx 0_{(D-k)\times k}$, and $\mathbf{k}_\theta(\overline{\mathbf{a}}_T, \overline{\mathbf{a}}_S) \approx 0_{k\times (D-k)}$. From Equations (20a) and (20b), then, it is evident that Algorithm 2 fails to utilize $\mathcal{D}_T$ well, if the set has limited space relevance.

In some embodiments, to ensure that both space- and time-relevant samples in $\mathcal{D}$ are able to contribute, a "spatiotemporal" JITL (ST-JITL) approach is used, with the following spatiotemporal Raman model (e.g., as local model 132):

$$b_i = g(\mathbf{a}_i, t_i) + \epsilon_i, \qquad\qquad \text{Equation (26)}$$

where $g \colon \mathbb{R}^{n_a} \times \mathbb{N} \to \mathbb{R}$ is the spatiotemporal Raman model and $t_i$ is the sample number of $\mathbf{a}_i$, and $\varepsilon_i \sim \mathcal{N}(0, \sigma^2)$ is a sequence of independent Gaussian random variables with zero mean and unknown variance $\sigma^2 \in \mathbb{R}_+$. In contrast to Equation (1), the spatiotemporal model of Equation (26) depends on both the spectral signal and its sampling time. As above, it is assumed that $g$ is a latent function modeled as a Gaussian process, such that for any input $(\mathbf{a}, t)$,

$$g(\mathbf{a}, t) \sim \mathcal{GP}\big(0, r_\theta(\mathbf{a}, \mathbf{a}, t, t)\big), \qquad\qquad \text{Equation (27)}$$

is a random function. For convenience, the mean function in Equation (27) is assumed to be zero, but this need not be the case in general. Further, for any arbitrary inputs $(\mathbf{a}_i, t_i)$ and $(\mathbf{a}_j, t_j)$, the covariance function $r_\theta(\mathbf{a}_i \mathbf{a}_j t_i t_j)$ can be defined as follows:

$$r_\theta\big(\mathbf{a}_i \mathbf{a}_j t_i t_j\big) = k_{\text{space}}\big(\mathbf{a}_i, \mathbf{a}_j\big) + k_{\text{time}}\big(t_i, t_j\big), \qquad\qquad \text{Equation (28)}$$

where $k_{\text{space}}\big(\mathbf{a}_i, \mathbf{a}_j\big) \in \mathbb{R}_+$ and $k_{\text{time}}\big(t_i, t_j\big) \in \mathbb{R}_+$ are the space covariance and time covariance between $(g(\mathbf{a}_i, t_i), g(\mathbf{a}_j, t_j))$, respectively. It is noted that, for a query $(\mathbf{a}^*, t^*)$, if a sample $(\mathbf{a}_j, b_j) \in \mathcal{D}_T$ has negligible space relevance then $k_{\text{space}}(\mathbf{a}_j, \mathbf{a}^*) \approx 0$ but $k_{\text{time}}(t_j, t^*) > 0$, such that Equation (28) defines a non-zero correlation between $(g(\mathbf{a}^*, t^*), g(\mathbf{a}_j, t_j))$. Finally, it should be noted that Equation (28) is a valid covariance function because the sum of two independent kernels is also a kernel. It is assumed that $k_{\text{space}}$ and $k_{\text{time}}$ are Gaussian kernels, such that for any input pair $(\mathbf{a}_i, t_i)$ and $(\mathbf{a}_j, t_j)$,

$$k_{\text{space}}\big(\mathbf{a}_i, \mathbf{a}_j\big) = \alpha_1 \exp - \left(\frac{\|\mathbf{a}_i - \mathbf{a}_j\|^2}{2\alpha_2}\right), \qquad\qquad \text{Equation (29a)}$$

$$k_{\text{time}}\big(a_i, t_j\big) = \beta_1 \exp - \left(\frac{\|t_i - t_j\|^2}{2\beta_2}\right), \qquad\qquad \text{Equation (29b)}$$

where $\theta \equiv [\alpha_1, \alpha_2, \beta_1, \beta_2]^T \in \Theta \in \mathbb{R}^4$ is the kernel parameter. Given Equations (29a) and (29b), Equation (28) ascribes a high correlation between $(g(\mathbf{a}_i, t_i), g(\mathbf{a}_j, t_j))$ if $(\mathbf{a}_i, t_i)$, $(\mathbf{a}_j, t_j)$ are close to each other. If $\overline{\mathbf{t}}_S = [t_1, \dots t_{D-k}]^T$ and $\overline{\mathbf{t}}_T = [t_{D-k+1}, \dots t_D]^T$ denote the sample numbers for the state and time relevant samples in $\mathcal{D}$, respectively, such that $\overline{\mathbf{t}} = [\overline{\mathbf{t}}_S; \overline{\mathbf{t}}_T]$, then for a query $(\mathbf{a}^*, t^*)$ the covariance function $r_\theta$ in Equation (28) can be written as

$$\mathbf{r}_\theta(\overline{\mathbf{a}}, \overline{\mathbf{a}}, \overline{\mathbf{t}}, \overline{\mathbf{t}}) \equiv \begin{bmatrix} \mathbf{r}_\theta(\overline{\mathbf{a}}_S, \overline{\mathbf{a}}_S, \overline{\mathbf{t}}_S, \overline{\mathbf{t}}_S) & \mathbf{r}_\theta(\overline{\mathbf{a}}_S, \overline{\mathbf{a}}_T, \overline{\mathbf{t}}_S, \overline{\mathbf{t}}_T) \\ \mathbf{r}_\theta(\overline{\mathbf{a}}_T, \overline{\mathbf{a}}_S, \overline{\mathbf{t}}_T, \overline{\mathbf{t}}_S) & \mathbf{r}_\theta(\overline{\mathbf{a}}_T, \overline{\mathbf{a}}_T, \overline{\mathbf{t}}_T, \overline{\mathbf{t}}_T) \end{bmatrix}, \qquad \text{Equation (30a)}$$

$$\mathbf{r}_\theta(\mathbf{a}^*, \overline{\mathbf{a}}, t^*, \overline{\mathbf{t}}) \equiv [\mathbf{r}_\theta(\mathbf{a}^*, \overline{\mathbf{a}}_S, t^*, \overline{\mathbf{t}}_S) \quad \mathbf{r}_\theta(\mathbf{a}^*, \overline{\mathbf{a}}_T, t^*, \overline{\mathbf{t}}_T)]. \qquad\qquad \text{Equation (30b)}$$

It is noted that, unlike variables a and b, the role of $t$ in Equations (30a) and (30b) is simply to improve the contribution of $\mathcal{D}_T$. Physically, given a, variable $t$ has not influence on b. Therefore, if $\overline{\mathbf{t}}_T = [t_{D-k+1}, \dots t_D]^T$ is defined as the sample number corresponding to samples in $\mathcal{D}_T$, $\overline{\mathbf{t}}_S = [t_1, \dots t_{D-k}]^T$ can be defined such that it satisfies the following:

$$|t_i - t_j| \gg M, \qquad \text{Equation (31a)}$$

$$|t_i - t^*| \gg N, \qquad \text{Equation (31b)}$$

$$|t_i - t_k| \gg P, \qquad \text{Equation (31c)}$$

for all $i, j \in \{1, ..., D - k\}$ and $k \in \{D - k + 1, ..., D\}$, where $M, N, P \in \mathbb{R}_+$ are arbitrary, large positive constants. Further, if it is assumed that $\bar{\mathbf{t}}_T$ and $t^*$ are such that $k_{\text{time}}(\bar{\mathbf{t}}_T, \bar{\mathbf{t}}_T > 0)$ and $k_{\text{time}}(t^*, \bar{\mathbf{t}}_T > 0)$, then for $\bar{\mathbf{t}}_T$ and $\bar{\mathbf{t}}_S$ as described above, $\mathbf{r}_\theta(\bar{\mathbf{a}}_S, \bar{\mathbf{a}}_S, \bar{\mathbf{t}}_S, \bar{\mathbf{t}}_S)$ can be written as follows:

$$\mathbf{r}_\theta(\bar{\mathbf{a}}_S, \bar{\mathbf{a}}_S, \bar{\mathbf{t}}_S, \bar{\mathbf{t}}_S) = \mathbf{k}_{\text{space}}(\bar{\mathbf{a}}_S, \bar{\mathbf{a}}_S) + \mathbf{k}_{\text{time}}(\bar{\mathbf{t}}_S, \bar{\mathbf{t}}_S), \qquad \text{Equation (32a)}$$

$$\approx \mathbf{k}_{\text{space}}(\bar{\mathbf{a}}_S, \bar{\mathbf{a}}_S) + \beta_1 \mathbf{I}_{(D-k)}, \qquad \text{Equation (32b)}$$

where Equation (32b) is from Equation (31a), which leads the off-diagonal entries in $k_{\text{time}}(\bar{\mathbf{t}}_S, \bar{\mathbf{t}}_S)$ to zero. Similarly, the covariance $\mathbf{r}_\theta(\mathbf{a}^*, \bar{\mathbf{a}}_S, t^*, \bar{\mathbf{t}}_S)$ and $\mathbf{r}_\theta(\mathbf{a}_S, \bar{\mathbf{a}}_T, \bar{\mathbf{t}}_S, \bar{\mathbf{t}}_T)$ can be computed as follows:

$$\mathbf{r}_\theta(\mathbf{a}^*, \bar{\mathbf{a}}_S, t^*, \bar{\mathbf{t}}_S) = \mathbf{k}_{\text{space}}(\mathbf{a}^*, \bar{\mathbf{a}}_S) + \mathbf{k}_{\text{time}}(t^*, \bar{\mathbf{t}}_S), \qquad \text{Equation (33a)}$$

$$\approx \mathbf{k}_{\text{space}}(\mathbf{a}^*, \bar{\mathbf{a}}_S), \qquad \text{Equation (33b)}$$

$$\mathbf{r}_\theta(\bar{\mathbf{a}}_S, \bar{\mathbf{a}}_T, \bar{\mathbf{t}}_S, \bar{\mathbf{t}}_T) = \mathbf{k}_{\text{space}}(\bar{\mathbf{a}}_S, \bar{\mathbf{a}}_T) + \mathbf{k}_{\text{time}}(\bar{\mathbf{t}}_S, \bar{\mathbf{t}}_T), \qquad \text{Equation (33c)}$$

$$\approx \mathbf{k}_{\text{space}}(\bar{\mathbf{a}}_S, \bar{\mathbf{a}}_T), \qquad \text{Equation (33d)}$$

where Equation (33b) is based on Equation (31b) and Equation (33d) is based on Equation (31c). Substituting Equations (32b), (33b) and (33d) into Equations (30a) and (30b) yields:

$$\mathbf{r}_\theta(\bar{\mathbf{a}}, \bar{\mathbf{a}}, \bar{\mathbf{t}}, \bar{\mathbf{t}}) = \begin{bmatrix} \mathbf{k}_{\text{space}}(\bar{\mathbf{a}}_S, \bar{\mathbf{a}}_S) + \beta_1 \mathbf{I}_{(D-k)} & \mathbf{k}_{\text{space}}(\bar{\mathbf{a}}_S, \bar{\mathbf{a}}_T) \\ \mathbf{k}_{\text{space}}(\bar{\mathbf{a}}_T, \bar{\mathbf{a}}_S) & \mathbf{r}_\theta(\bar{\mathbf{a}}_T, \bar{\mathbf{a}}_T, \bar{\mathbf{t}}_T, \bar{\mathbf{t}}_T) \end{bmatrix}, \qquad \text{Equation (34a)}$$

$$\mathbf{r}_\theta(\mathbf{a}^*, \bar{\mathbf{a}}, t^*, \bar{\mathbf{t}}) = [\mathbf{k}_{\text{space}}(\mathbf{a}^*, \bar{\mathbf{a}}_S) \quad \mathbf{r}_\theta(\mathbf{a}^*, \bar{\mathbf{a}}_T, t^*, \bar{\mathbf{t}}_T)]. \qquad \text{Equation (34b)}$$

From Equations (30a) and (30b), it is straightforward to confirm that the covariance $\mathbf{r}_\theta$ includes contributions from both $\mathbf{k}_{\text{space}}$ and $\mathbf{k}_{\text{time}}$. Given covariance functions for the spatiotemporal Raman model in Equations (30a) and (30b), the kernel parameter $\theta$ and the noise variance $\sigma^2$ can be estimated by maximizing

$$\log p(\bar{\mathbf{b}}|\bar{\mathbf{a}}, \bar{\mathbf{t}}) = -\tfrac{1}{2}\bar{\mathbf{b}}^{\text{T}}\mathbf{r}_\gamma^{-1}\bar{\mathbf{b}} - \tfrac{1}{2}\log|\mathbf{r}_\gamma| - \tfrac{D}{2}\log 2\pi, \qquad \text{Equation (35)}$$

where $\gamma = [\theta, \sigma^2]^{\text{T}} \in \Gamma \, \mathbb{R}^5$, $\log p(\bar{\mathbf{b}}|\bar{\mathbf{a}}, \bar{\mathbf{t}})$ is the log marginalized likelihood function, and $\mathbf{r}_\gamma = \mathbf{r}_\theta + \mathbf{I}_{D \times D}$. Maximizing Equation (35) over $\Gamma$ yields an optimal estimate, $\gamma^*$. For gradient-based optimizers, gradients for Equation (35) with respect to $\gamma$ can be computed in a manner similar to Equation (10b). Given y*, the point estimate and the posterior variance for a query $(\mathbf{a}^*, t^*)$ can be computed as

$$\hat{b} = \mathbf{r}_\theta(\mathbf{a}^*, \bar{\mathbf{a}}, t^*, \bar{\mathbf{t}})\left[\mathbf{r}_\gamma(\bar{\mathbf{a}}, \bar{\mathbf{a}}, \bar{\mathbf{t}}, \bar{\mathbf{t}})\right]^{-1}\bar{\mathbf{b}}, \qquad \text{Equation (36a)}$$

$$r_\theta^* = r_\theta(\mathbf{a}^*, \mathbf{a}^*, t^*, t^*) - \mathbf{r}_\theta(\mathbf{a}^*, \bar{\mathbf{a}}, t^*, \bar{\mathbf{t}})\left[\mathbf{r}_\gamma(\bar{\mathbf{a}}, \bar{\mathbf{a}}, \bar{\mathbf{t}}, \bar{\mathbf{t}})\right]^{-1}\text{x } \mathbf{r}_\theta(\bar{\mathbf{a}}, \mathbf{a}^*, \bar{\mathbf{t}}, t^*), \qquad \text{Equation (36b)}$$

where the covariance functions are given in Equations (34a) and (34b). Similarly, the credibility bounds ($b^L \le \hat{b} \le b^U$) on the point-estimate in Equation (36a) can be computed as follows:

$$b^L = \hat{b} - 2\sqrt{r_\gamma^*},$$

Equation (37a)

$$b^U = \hat{b} + 2\sqrt{r_\gamma^*},$$

Equation (37b)

where $r_\gamma^* = r_\theta^* + \sigma^2$. From Equations (36a), (37a) and (37b), it is straightforward to see that both space- and time-relevant samples contribute to the model prediction and credibility bound calculations. Finally, substituting Equations (34a) and (34b) into Equations (36a) and (36b) yields the posterior mean and variance, respectively. It should be noted that, unlike in the case of Algorithm 2, the model prediction in Equation (36a), and the credibility intervals in Equations (37a) and (37b), depend on $\mathcal{D}_T$ even when $\mathcal{D}_T$ has no space relevance. For example, when $\mathcal{D}_T$ has no space relevance (i.e., $\mathbf{k}_{\mathrm{space}}(\bar{\mathbf{a}}_S, \bar{\mathbf{a}}_T) \approx 0_{(D-k)\mathbf{x}k}$ and $\mathbf{k}_{\mathrm{space}}(\mathbf{a}^*, \bar{\mathbf{a}}_T) \approx 0_{\mathbf{1x}k}$), then Equations (36a) and (36b) can be written as:

$$\mathbf{r}_\theta(\bar{\mathbf{a}}, \bar{\mathbf{a}}, \bar{\mathbf{t}}, \bar{\mathbf{t}}) = \begin{bmatrix} \mathbf{k}_{\mathrm{space}}(\bar{\mathbf{a}}_S, \bar{\mathbf{a}}_S) + \beta_1 \mathbf{I}_{(D-k)} & 0_{(D-k)\mathbf{x}k} \\ 0_{k\mathbf{x}(D-k)} & \mathbf{r}_\theta(\bar{\mathbf{a}}_T, \bar{\mathbf{a}}_T, \bar{\mathbf{t}}_T, \bar{\mathbf{t}}_T) \end{bmatrix},$$

Equation (38a)

$$\mathbf{r}_\theta(\mathbf{a}^*, \bar{\mathbf{a}}, t^*, \bar{\mathbf{t}}) = [\mathbf{k}_{\mathrm{space}}(\mathbf{a}^*, \bar{\mathbf{a}}_S) \quad \mathbf{k}_{\mathrm{time}}(t^*, \bar{\mathbf{t}}_T)].$$

Equation (38b)

It can be seen from the above that Equations (38a) and (38b) still include contributions from both $\mathbf{k}_{\mathrm{space}}$ and $\mathbf{k}_{\mathrm{time}}$. An example algorithm that formally outlines the ST-JITL technique is provided below in Algorithm 3:

1.  **Input:** Library $\mathcal{L}_t = \{(\mathbf{a}_i, \mathrm{b}_i)\}_{i=1}^{L}$, query point $\mathbf{a}^*$

2.  **Output:** Prediction $\hat{b}$ and uncertainty $(b^L, b^U)$

3.  Set $\mathcal{K} \leftarrow \{\varnothing\}$ and $\bar{\mathbf{t}}_T \leftarrow \{\varnothing\}$

4.  **for** $t = 1$ to $T$ **do**

5.      Set $I \leftarrow \mathrm{sample\_index}(\mathcal{L}_t)$, $\mathcal{D}_S \leftarrow \{\varnothing\}$, $\mathcal{D}_T \leftarrow \{\varnothing\}$

6.      **for** $d = 1$ to $D - \mathrm{set\_cardinality}(\mathcal{K})$ **do**

7.          $i_* \in \arg\max_{i \in I} \mathrm{sim}(\mathbf{a}_i, \mathbf{a}^*)$

8.          $\mathcal{D}_S \leftarrow \mathcal{D}_S \cup \{\mathbf{a}_{i_*}, \mathrm{b}_{i_*}\}$

9.          $I \leftarrow I \setminus \{i_*\}$

10.     **end for**

11.     **if** $\mathrm{set\_cardinality}(\mathcal{K}) \geq 1$ **then**

12.         $\mathcal{D}_T \leftarrow \mathcal{K}$

13.     **end if**

14.     $\mathcal{D} \leftarrow \mathcal{D}_S \cup \mathcal{D}_T$

15.     Set $\bar{\mathbf{t}}_S$ according to Equations (31a) through (31c)

16.     Set $\bar{\mathbf{t}} \leftarrow [\bar{\mathbf{t}}_S; \bar{\mathbf{t}}_T]$

17.     Train Gaussian process model in Equation (28) using $\mathcal{D}$ and $\bar{\mathbf{t}}$ and estimate $\gamma^*$

18.     Compute $\hat{b}$ using Equation (36a), and compute $(b^L, b^U)$ using Equations (37a) and (37b)

19.     **if** $b^*$ is available **then**

20.         **if** $\mathrm{size}(\mathcal{K}) = k$ **then**

21.             $\mathcal{L}_t \leftarrow \mathcal{L}_t \cup \mathrm{select\_oldest}(\mathcal{K})$

22.             $\mathcal{K} \leftarrow \mathrm{delete\_oldest}(\mathcal{K})$

23.             $\mathcal{K} \leftarrow \mathcal{K} \cup \{\mathbf{a}^*, b^*\}$

24.         **end if**

25.         $\mathcal{K} \leftarrow \mathcal{K} \cup \{\mathbf{a}^*, b^*\}$

26.     **end if**

27. **end for**

**Algorithm 3**

It is noted that A-JITL and ST-JITL (in Algorithms 2 and 3, respectively) can be identical for the case where $\beta_1 = 0$. This is because, for $\beta_1 = 0$, $k_{\mathrm{time}} = 0$ such that $r_\theta = k_{\mathrm{space}} = k_\theta$ (as seen from Equations (28) and (29b)).

Turning now to FIG. 6, an example data flow 350 that may occur when analyzing a biopharmaceutical process using an ST-JITL technique as described herein is shown. The data flow 350 may occur within system 100 of FIG. 1 or system 150 of FIG. 2, for example. In the data flow 350, spectral data 352 is provided by a spectrometer/probe. For example, spectral data 352 may include a Raman scan vector generated by Raman analyzer 106, or an NIR scan vector, etc. A query point 354 is generated (e.g., by query unit 140) based on spectral data 352, and is used to query a global data set 356, which may include all of the observation data sets in observation database 136, for example. Global data set 356 is logically separated into the last $k$ entries 357A (e.g., all from the current experiment/process), and all entries 357B prior to the last k entries 357A (e.g., from previous, and possibly also the current, experiment/process). The value of $k$ may be determined based on the sample number of the query point 354. Local data set 358 may be generated from entries 357A and entries 357B in accordance with Algorithm 3, for example.

Local data set 358 is then used as training data (e.g., by local model generator 142) to calibrate a local model 360 (e.g., local model 132). Local model 360 is then used (e.g., by prediction unit 144) to predict an output (analytical measurement) 362, such as a media component concentration, media state (e.g., glucose, lactate, glutamate, glutamine, ammonia, amino acids, Na+, K+ and other nutrients or metabolites, pH, $pCO_2$, $pO_2$, temperature, osmolality, etc.), viable cell density, titer, critical quality attributes, cell state, etc., and possibly also output credibility bounds or another suitable confidence indicator.

If an actual analytical measurement (e.g., a measurement made by an analytical instrument such as one of analytical instrument(s) 104) is available, a new entry 364 (including the sample number thereof) is created and added to global data set 356. Such measurements may be available on a periodic sampling basis (e.g., once or twice per day), for example, and/or may be made available in response to a trigger with variable timing (e.g., if a certain number of predictions in a row have unacceptably wide credibility bounds, etc.).

As noted above, analytical measurements may be scheduled/triggered based on the current and/or recent performance of one or more local models (e.g., local model 132, 260, 310, or 360), in order to maintain or improve prediction accuracy while reducing resource usage (e.g., usage of analytical instruments). This technique may be used with A-JITL, ST-JITL, or straight JITL, for example.

In one embodiment, credibility intervals are used to trigger model maintenance. In particular, if the width of the credibility interval (e.g., the distance between credibility bounds as computed using Equation (16) or Equations (37a), (37b)) around a given model prediction (e.g., around the most recent prediction made by local model 132, 260, 310, or 360) is greater than a pre-defined threshold, database maintenance unit 146 may generate a request message, and cause computer 110 to send the message to analytical instrument(s) 104 to request a measurement. In the example results of FIG. 3, for instance, database maintenance unit 146 might trigger new analytical measurements near the end of days 12/08/17, 12/09/17, and 12/14/17, where shaded areas 208 indicate a wide credibility interval (i.e., a large value of $b^U$ - $b^L$).

In response to the request message, analytical measurement(s) 104 perform(s) the measurement(s), and provide the measurement(s) to computer 110. Database maintenance unit 146 may then send the measurement(s), and the corresponding Raman scan vector(s) received from Raman analyzer 106, to database server 112 for storage in observation database 136. For example, the measurement(s) and scan vector(s) may be added to the library $\mathcal{L}$ (for straight JITL) or the library $\mathcal{K}$ (for A-JITL or ST-JITL) discussed above.

Conversely, if the width of the credibility interval around a given model prediction is not greater than the pre-defined threshold, database maintenance unit 146 may not request a new analytical measurement, in which case the library in observation database 136 remains unchanged. In embodiments where analytical instrument(s) 104 includes multiple instruments measuring different properties such as media component concentration, media state (e.g., glucose, lactate, glutamate, glutamine, ammonia, amino acids, Na+, K+ and other nutrients or metabolites, pH, $pCO_2$, $pO_2$, temperature, osmolality, etc.), viable cell density, titer, critical quality attributes, cell state, etc., and separate local models are used to predict different the various property values, the scheduling process may be implemented separately for each predicted property and the analytical instrument that measures that property, possibly with different credibility interval width thresholds for each property.

Mathematically, database maintenance unit 146 may schedule/trigger the new analytical measurement(s) at a query point a* under the condition:

$$b^U - b^L \geq THR ,\qquad\qquad\qquad\text{Equation (39)}$$

where *THR* is the user-defined threshold. In some embodiments, *THR* may be adjusted by a user to suit a particular application or use case. For example, a user may set a relatively small *THR* value (used by database maintenance unit 146) for an application where model reliability is critical, thereby causing the model/library maintenance operations to occur more frequently. In general, *THR* may be set to different values based on process criticality, based on the parameter being predicted such as media component concentration, media state (e.g., glucose, lactate, glutamate, glutamine, ammonia, amino acids, Na+, K+ and other nutrients or metabolites, pH, $pCO_2$, $pO_2$, temperature, osmolality, etc.), viable cell density, titer, critical quality attributes, cell state, etc., and/or based on the current time period (e.g., using a lower *THR* for later days of a culture as compared to the initial days). The selection of *THR* represents a trade-off between model accuracy and resource (analytical instrument) usage, with lower thresholds tending to increase model accuracy at the expense of increased resource usage.

Variations of this scheduling protocol are also possible. In one embodiment, for example, database maintenance unit 146 may apply one or more model performance criteria to not only the current (most recent) prediction, but also one or more other, recent predictions (e.g., the most recent N predictions, where *N* > 1). As an example of such an embodiment, database maintenance unit 146 may compute an average width of the credibility intervals for the most recent N predictions ($N \geq 1$), and then compare that average width to the threshold *THR*. As another example, database maintenance unit 146 may identify the *X* largest credibility interval widths among the last *Y* predictions (*X* < *Y*), and schedule/trigger a new analytical measurement only if each of those *X* widths is greater than the threshold *THR*.

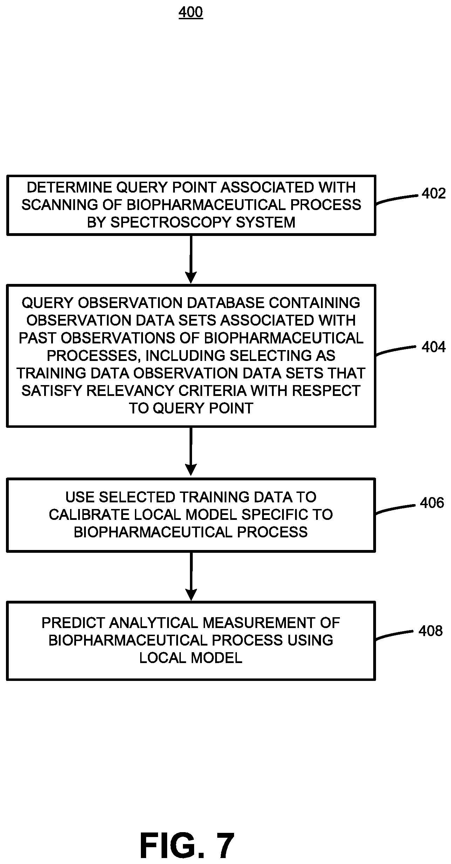

FIG. 7 is a flow diagram of an example method 400 for analyzing a biopharmaceutical process (e.g., for monitoring and/or control purposes). The method 400 may be implemented by a computer such as computer 110 of FIG. 1 (e.g., by processing unit 120 executing instructions of JITL predictor application 130) or FIG. 2, and/or by a server such as database server 112 of FIG. 1 or FIG. 2, for example.

At block 402, a query point that is associated with the scanning of a biopharmaceutical process by a spectroscopy system (e.g., by Raman analyzer 104 and Raman probe 106 of system 100 or system 150) is determined. The query point may be determined based at least in part on a spectral scan vector (e.g., a Raman or NIR scan vector) that was generated by the spectroscopy system when scanning the biopharmaceutical process, for example. Depending on the embodiment, the query point may be determined based on the raw spectral scan vector, or after suitable pre-processing filtering of the raw spectral scan vector. In some embodiments, the query point is also determined based on other information, such as a media profile associated with the biopharmaceutical process (e.g., a fluid type, specific nutrients, a pH level, etc.), and/or one or more operating conditions under which the biopharmaceutical process is analyzed (e.g., a metabolite concentration set point, etc.), for example.

At block 404, an observation database (e.g., observation database 136) is queried. The observation database may contain observation data sets associated with past observations of a number of biopharmaceutical processes. Each of the observation data sets may include spectral data (e.g., a Raman or NIR scan vector) and a corresponding analytical measurement (or, in some embodiments, two or more analytical measurements). The analytical measurement may be a media component concentration, media state (e.g., glucose, lactate, glutamate, glutamine, ammonia, amino acids, Na+, K+ and other nutrients or metabolites, pH, $pCO_2$, $pO_2$, temperature, osmolality, etc.), viable cell density, titer, critical quality attributes, and/or cell state, for example.

Block 404 may include selecting as training data, from among the observation data sets, those observation data sets that satisfy one or more relevancy criteria with respect to the query point. If the query point included a spectral scan vector, for example, block 404 may include comparing that spectral scan vector to the spectral scan vectors associated with each of the past observations represented in the observation database (e.g., by calculating Euclidean or other distances between (1) the spectral scan vector on which determination of the query point was based and (2) each of the spectral scan vectors associated with the past observations, and then selecting as the training data any of the spectral scan vectors associated with past observations that are determined to be within a threshold distance of the spectral scan vector on which determination of the query point was based).

At block 406, the selected training data is used to calibrate a local model that is specific to the biopharmaceutical process being monitored. The local model (e.g., local model 132) is trained, at block 406, to predict analytical measurements based on spectral data inputs (e.g., Raman or NIR spectral scan vectors). In some embodiments, the local model is a Gaussian process machine-learning model.

At block 408, an analytical measurement of the biopharmaceutical process is predicted using the local model. Block 408 may include using the local model to analyze spectral data (e.g., a Raman or NIR scan vector) that the spectroscopy system generated when scanning the biopharmaceutical process. For example, block 408 may include predicting the analytical measurement by using the local model to process the same scan vector or other spectral data on which the query point was based. Depending on the embodiment, the local model may be used to analyze the raw spectral data (e.g., a raw Raman scan vector), or to analyze the spectral data after suitable pre-processing filtering of the raw spectral data. In some embodiments, block 408 also includes determining a confidence indicator (e.g., credibility bounds, a confidence score, etc.) associated with the predicted analytical measurement of the biopharmaceutical process. In some embodiments, the local model also predicts one or more additional analytical measurements at block 408.

In some embodiments, method 400 includes one or more additional blocks not shown in FIG. 5. For example, method 400 may include an additional block in which at least one parameter of the biopharmaceutical process is controlled, based at least in part on the analytical measurement predicted at block 408. Depending on the embodiment, the parameter may be of the same type as the predicted analytical measurement (e.g., controlling a glucose concentration based on a predicted glucose concentration), or of a different type. Model predictive control (MPC) techniques may be used to control the parameter (or parameters), for example.

As another example, method 400 may include a first additional block in which an actual analytical measurement of the biopharmaceutical process is obtained (e.g., by or from one of analytical instrument(s) 104, in response to determining that the predicted analytical measurement, and possibly also one or more earlier/recent measurements, do/does not satisfy one or more model performance criteria, as discussed above), and a second additional block in which (1) spectral data that the spectroscopy system generated when the actual analytical measurement was obtained, and (2) the actual analytical measurement of the biopharmaceutical process, are caused to be added to the observation database (e.g., by sending the spectral data and analytical measurement to a database server such as database server 112, or by directly adding the spectral data and analytical measurement to a local observation database, etc.). In embodiments where multiple types of analytical measurements are predicted, multiple actual analytical measurements may be obtained and added to the observation database.

As yet another example, method 400 may include one or more additional sets of blocks, each similar to blocks 402 through 408. In each of these additional sets of blocks, a local model may be calibrated by querying the observation database (or another observation database), and used to predict a different type of analytical measurement.

Additional considerations pertaining to this disclosure will now be addressed.

The terms "polypeptide" or "protein" are used interchangeably throughout and refer to a molecule comprising two or more amino acid residues joined to each other by peptide bonds. Polypeptides and proteins also include macromolecules having one or more deletions from, insertions to, and/or substitutions of the amino acid residues of the native sequence, that is, a polypeptide or protein produced by a naturally-occurring and non-recombinant cell; or is produced by a genetically-engineered or recombinant cell, and comprise molecules having one or more deletions from, insertions to, and/or substitutions of the amino acid residues of the amino acid sequence of the native protein. Polypeptides and proteins also include amino acid polymers in which one or more amino acids are chemical analogs of a corresponding naturally-occurring amino acid and polymers. Polypeptides and proteins are also inclusive of modifications including, but not limited to, glycosylation, lipid attachment, sulfation, gamma-carboxylation of glutamic acid residues, hydroxylation and ADP-ribosylation.

Polypeptides and proteins can be of scientific or commercial interest, including protein-based therapeutics. Proteins include, among other things, secreted proteins, non-secreted proteins, intracellular proteins or membrane-bound proteins. Polypeptides and proteins can be produced by recombinant animal cell lines using cell culture methods and may be referred to as "recombinant proteins". The expressed protein(s) may be produced intracellularly or secreted into the culture medium from which it can be recovered and/or collected. Proteins include proteins that exert a therapeutic effect by binding a target, particularly a target among those listed below, including targets derived therefrom, targets related thereto, and modifications thereof.

Proteins "antigen-binding proteins". Antigen-binding protein refers to proteins or polypeptides that comprise an antigen-binding region or antigen-binding portion that has a strong affinity for another molecule to which it binds (antigen). Antigen-binding proteins encompass antibodies, peptibodies, antibody fragments, antibody derivatives, antibody analogs, fusion proteins (including single-chain variable fragments (scFvs) and double-chain (divalent) scFvs, muteins, xMAbs, and chimeric antigen receptors (CARs).

An scFv is a single chain antibody fragment having the variable regions of the heavy and light chains of an antibody linked together. See U.S. Patent Nos. 7,741,465, and 6,319,494 as well as Eshhar et al., Cancer Immunol Immunotherapy (1997) 45: 131-136. An scFv retains the parent antibody's ability to specifically interact with target antigen.

The term "antibody" includes reference to both glycosylated and non-glycosylated immunoglobulins of any isotype or subclass or to an antigen-binding region thereof that competes with the intact antibody for specific binding. Unless otherwise specified, antibodies include human, humanized, chimeric, multi-specific, monoclonal, polyclonal, heterolgG, XmAbs, bispecific, and oligomers or antigen binding fragments thereof. Antibodies include the IgG1-, IgG2-IgG3- or IgG4-type. Also included are proteins having an antigen binding fragment or region such as Fab, Fab', F(ab')2, Fv, diabodies, Fd, dAb, maxibodies, single chain antibody molecules, single domain VHH, complementarity determining region (CDR) fragments, scFv, diabodies, triabodies, tetrabodies and polypeptides that contain at least a portion of an immunoglobulin that is sufficient to confer specific antigen binding to a target polypeptide.

Also included are human, humanized, and other antigen-binding proteins, such as human and humanized antibodies, that do not engender significantly deleterious immune responses when administered to a human.

Also included are peptibodies, polypeptides comprising one or more bioactive peptides joined together, optionally via linkers, with an Fc domain. See U.S. Patent No. 6,660,843, U.S. Patent No. 7,138,370 and U.S. Patent No. 7,511,012.

Proteins also include genetically engineered receptors such as chimeric antigen receptors (CARs or CAR-Ts) and T cell receptors (TCRs). CARs typically incorporate an antigen binding domain (such as scFv) in tandem with one or more costimulatory ("signaling") domains and one or more activating domains.

Also included are bispecific T cell engagers (BiTE®) antibody constructs are recombinant protein constructs made from two flexibly linked antibody derived binding domains (see WO 99/54440 and WO 2005/040220). One binding domain of the construct is specific for a selected tumor- associated surface antigen on target cells; the second binding domain is specific for CD3, a subunit of the T cell receptor complex on T cells. The BiTE® constructs may also include the ability to bind to a context independent epitope at the N-terminus of the CD3s chain (WO 2008/119567) to more specifically activate T cells. Half-life extended BiTE® constructs include fusion of the small bispecific antibody construct to larger proteins, which preferably do not interfere with the therapeutic effect of the BiTE® antibody construct. Examples for such further developments of bispecific T cell engagers comprise bispecific Fc-molecules e.g. described in US 2014/0302037, US 2014/0308285, WO 2014/151910 and WO 2015/048272. An alternative strategy is the use of human serum albumin (HAS) fused to the bispecific molecule or the mere fusion of human albumin binding peptides (see e.g. WO 2013/128027, WO2014/140358). Another HLE BiTE® strategy comprises fusing a first domain binding to a target cell surface antigen, a second domain binding to an extracellular epitope of the human and/or the Macaca CD3e chain and a third domain, which is the specific Fc modality (WO 2017/134140).

Also included are modified proteins, such as are proteins modified chemically by a non-covalent bond, covalent bond, or both a covalent and non-covalent bond. Also included are proteins further comprising one or more post-translational modifications which may be made by cellular modification systems or modifications introduced *ex vivo* by enzymatic and/or chemical methods or introduced in other ways.

Proteins may also include recombinant fusion proteins comprising, for example, a multimerization domain, such as a leucine zipper, a coiled coil, an Fc portion of an immunoglobulin, and the like. Also included are proteins comprising all or part of the amino acid sequences of differentiation antigens (referred to as CD proteins) or their ligands or proteins substantially similar to either of these.

In some embodiments, proteins may include colony stimulating factors, such as granulocyte colony-stimulating factor (G-CSF). Such G-CSF agents include, but are not limited to, Neupogen® (filgrastim) and Neulasta® (pegfilgrastim). Also included are erythropoiesis stimulating agents (ESA), such as Epogen® (epoetin alfa), Aranesp® (darbepoetin alfa), Dynepo® (epoetin delta), Mircera® (methyoxy polyethylene glycol-epoetin beta), Hematide®, MRK-2578, INS-22, Retacrit® (epoetin zeta), Neorecormon® (epoetin beta), Silapo® (epoetin zeta), Binocrit® (epoetin alfa), epoetin alfa Hexal, Abseamed® (epoetin alfa), Ratioepo® (epoetin theta), Eporatio® (epoetin theta), Biopoin® (epoetin theta), epoetin alfa, epoetin beta, epoetin zeta, epoetin theta, and epoetin delta, epoetin omega, epoetin iota, tissue plasminogen activator, GLP-1 receptor agonists, as well as the molecules or variants or analogs thereof and biosimilars of any of the foregoing.

In some embodiments, proteins may include proteins that bind specifically to one or more CD proteins, HER receptor family proteins, cell adhesion molecules, growth factors, nerve growth factors, fibroblast growth factors, transforming growth factors (TGF), insulin-like growth factors, osteoinductive factors, insulin and insulin-related proteins, coagulation and coagulation-related proteins, colony stimulating factors (CSFs), other blood and serum proteins blood group antigens; receptors, receptor-associated proteins, growth hormones, growth hormone receptors, T-cell receptors; neurotrophic factors, neurotrophins, relaxins, interferons, interleukins, viral antigens, lipoproteins, integrins, rheumatoid factors, immunotoxins, surface membrane proteins, transport proteins, homing receptors, addressins, regulatory proteins, and immunoadhesins.

In some embodiments proteins may include proteins that bind to one of more of the following, alone or in any combination: CD proteins including but not limited to CD3, CD4, CD5, CD7, CD8, CD19, CD20, CD22, CD25, CD30, CD33, CD34, CD38, CD40, CD70, CD123, CD133, CD138, CD171, and CD174, HER receptor family proteins, including, for instance, HER2, HER3, HER4, and the EGF receptor, EGFRvIII, cell adhesion molecules, for example, LFA-1, Mol, p150,95, VLA-4, ICAM-1, VCAM, and alpha v/beta 3 integrin, growth factors, including but not limited to, for example, vascular endothelial growth factor ("VEGF"); VEGFR2, growth hormone, thyroid stimulating hormone, follicle stimulating hormone, luteinizing hormone, growth hormone releasing factor, parathyroid hormone, mullerian-inhibiting substance, human macrophage inflammatory protein (MIP-1-alpha), erythropoietin (EPO), nerve growth factor, such as NGF-beta, platelet-derived growth factor (PDGF), fibroblast growth factors, including, for instance, aFGF and bFGF, epidermal growth factor (EGF), Cripto, transforming growth factors (TGF), including, among others, TGF-$\alpha$ and TGF-$\beta$, including TGF-$\beta$1, TGF-$\beta$2, TGF-$\beta$3, TGF-$\beta$4, or TGF-$\beta$5, insulin-like growth factors-I and -II (IGF-I and IGF-II), des(1-3)-IGF-I (brain IGF-I), and osteoinductive factors, insulins and insulin-related proteins, including but not limited to insulin, insulin A-chain, insulin B-chain, proinsulin, and insulin-like growth factor binding proteins; (coagulation and coagulation-related proteins, such as, among others, factor VIII, tissue factor, von Willebrand factor, protein C, alpha-1-antitrypsin, plasminogen activators, such as urokinase and tissue plasminogen activator ("t-PA"), bombazine, thrombin, thrombopoietin, and thrombopoietin receptor, colony stimulating factors (CSFs), including the following, among others, M-CSF, GM-CSF, and G-CSF, other blood and serum proteins, including but not limited to albumin, IgE, and blood group antigens, receptors and receptor-associated proteins, including, for example, flk2/flt3 receptor, obesity (OB) receptor, growth hormone receptors, and T-cell receptors; (x) neurotrophic factors, including but not limited to, bone-derived neurotrophic factor (BDNF) and neurotrophin-3, -4, -5, or -6 (NT-3, NT-4, NT-5, or NT-6); (xi) relaxin A-chain, relaxin B-chain, and prorelaxin, interferons, including for example, interferon-alpha, -beta, and -gamma, interleukins (ILs), e.g., IL-1 to IL-10, IL-12, IL-15, IL-17, IL-23, IL-12/IL-23, IL-2Ra, IL1-R1, IL-6 receptor, IL-4 receptor and/or IL-13 to the receptor, IL-13RA2, or IL-17 receptor, IL-1RAP,; (xiv) viral antigens, including but not limited to, an AIDS envelope viral antigen, lipoproteins, calcitonin, glucagon, atrial natriuretic factor, lung surfactant, tumor necrosis factor-alpha and -beta, enkephalinase, BCMA, IgKappa, ROR-1, ERBB2, mesothelin, RANTES (regulated on activation normally T-cell expressed and secreted), mouse gonadotropin-associated peptide, Dnase, FR-alpha, inhibin, and activin, integrin, protein A or D, rheumatoid factors, immunotoxins, bone morphogenetic protein (BMP), superoxide dismutase, surface membrane proteins, decay accelerating factor (DAF), AIDS envelope, transport proteins, homing receptors, MIC (MIC-a, MIC-B), ULBP 1-6, EPCAM, addressins, regulatory proteins, immunoadhesins, antigen-binding proteins, somatropin, CTGF, CTLA4, eotaxin-1, MUC1, CEA, c-MET, Claudin-18, GPC-3, EPHA2, FPA, LMP1, MG7, NY-ESO-1, PSCA, ganglioside GD2, glanglioside GM2, BAFF, OPGL (RANKL), myostatin, Dickkopf-1 (DKK-1), Ang2, NGF, IGF-1 receptor, hepatocyte growth factor (HGF), TRAIL-R2, c-Kit, B7RP-1, PSMA, NKG2D-1, programmed cell death protein 1 and ligand, PD1 and PDL1, mannose receptor/hCG$\beta$, hepatitis-C virus, mesothelin dsFv[PE38 conjugate, Legionella pneumophila (IIy), IFN gamma, interferon gamma induced protein 10 (IP10), IFNAR, TALL-1, thymic stromal lymphopoietin (TSLP), proprotein convertase subtilisin/Kexin Type 9 (PCSK9), stem cell factors, Flt-3, calcitonin gene-related peptide (CGRP), OX40L, $\alpha$4$\beta$7, platelet specific (platelet

glycoprotein IIb/IIIb (PAC-1), transforming growth factor beta (TFGβ), Zona pellucida sperm-binding protein 3 (ZP-3), TWEAK, platelet derived growth factor receptor alpha (PDGFRa), sclerostin, and biologically active fragments or variants of any of the foregoing.

In another embodiment, proteins include abciximab, adalimumab, adecatumumab, aflibercept, alemtuzumab, alirocumab, anakinra, atacicept, basiliximab, belimumab, bevacizumab, biosozumab, blinatumomab, brentuximab vedotin, brodalumab, cantuzumab mertansine, canakinumab, cetuximab, certolizumab pegol, conatumumab, daclizumab, denosumab, eculizumab, edrecolomab, efalizumab, epratuzumab, etanercept, evolocumab, galiximab, ganitumab, gemtuzumab, golimumab, ibritumomab tiuxetan, infliximab, ipilimumab, lerdelimumab, lumiliximab, lxdkizumab, mapatumumab, motesanib diphosphate, muromonab-CD3, natalizumab, nesiritide, nimotuzumab, nivolumab, ocrelizumab, ofatumumab, omalizumab, oprelvekin, palivizumab, panitumumab, pembrolizumab, pertuzumab, pexelizumab, ranibizumab, rilotumumab, rituximab, romiplostim, romosozumab, sargamostim, tocilizumab, tositumomab, trastuzumab, ustekinumab, vedolizumab, visilizumab, volociximab, zanolimumab, zalutumumab, and biosimilars of any of the foregoing.

Proteins encompass all of the foregoing and further include antibodies comprising 1, 2, 3, 4, 5, or 6 of the complementarity determining regions (CDRs) of any of the aforementioned antibodies. Also included are variants that comprise a region that is 70% or more, especially 80% or more, more especially 90% or more, yet more especially 95% or more, particularly 97% or more, more particularly 98% or more, yet more particularly 99% or more identical in amino acid sequence to a reference amino acid sequence of a protein of interest. Identity in this regard can be determined using a variety of well-known and readily available amino acid sequence analysis software. Preferred software includes those that implement the Smith-Waterman algorithms, considered a satisfactory solution to the problem of searching and aligning sequences. Other algorithms also may be employed, particularly where speed is an important consideration. Commonly employed programs for alignment and homology matching of DNAs, RNAs, and polypeptides that can be used in this regard include FASTA, TFASTA, BLASTN, BLASTP, BLASTX, TBLASTN, PROSRCH, BLAZE, and MPSRCH, the latter being an implementation of the Smith-Waterman algorithm for execution on massively parallel processors made by MasPar.

Some of the figures described herein illustrate example block diagrams having one or more functional components. It will be understood that such block diagrams are for illustrative purposes and the devices described and shown may have additional, fewer, or alternate components than those illustrated. Additionally, in various embodiments, the components (as well as the functionality provided by the respective components) may be associated with or otherwise integrated as part of any suitable components.

Embodiments of the disclosure relate to a non-transitory computer-readable storage medium having computer code thereon for performing various computer-implemented operations. The term "computer-readable storage medium" is used herein to include any medium that is capable of storing or encoding a sequence of instructions or computer codes for performing the operations, methodologies, and techniques described herein. The media and computer code may be those specially designed and constructed for the purposes of the embodiments of the disclosure, or they may be of the kind well known and available to those having skill in the computer software arts. Examples of computer-readable storage media include, but are not limited to: magnetic media such as hard disks, floppy disks, and magnetic tape; optical media such as CD-ROMs and holographic devices; magneto-optical media such as optical disks; and hardware devices that are specially configured to store and execute program code, such as ASICs, programmable logic devices ("PLDs"), and ROM and RAM devices.

Examples of computer code include machine code, such as produced by a compiler, and files containing higher-level code that are executed by a computer using an interpreter or a compiler. For example, an embodiment of the disclosure may be implemented using Java, C++, or other object-oriented programming language and development tools. Additional examples of computer code include encrypted code and compressed code. Moreover, an embodiment of the disclosure may be downloaded as a computer program product, which may be transferred from a remote computer (e.g., a server computer) to a requesting computer (e.g., a client computer or a different server computer) via a transmission channel. Another embodiment of the disclosure may be implemented in hardwired circuitry in place of, or in combination with, machine-executable software instructions.

As used herein, the singular terms "a," "an," and "the" may include plural referents, unless the context clearly dictates otherwise.

As used herein, the terms "connect," "connected," and "connection" refer to an operational coupling or linking. Connected components can be directly or indirectly coupled to one another, for example, through another set of components.

As used herein, the terms "approximately," "substantially," "substantial" and "about" are used to describe and account for small variations. When used in conjunction with an event or circumstance, the terms can refer to instances in which the event or circumstance occurs precisely as well as instances in which the event or circumstance occurs to a close approximation. For example, when used in conjunction with a numerical value, the terms can refer to a range of variation less than or equal to ±10% of that numerical value, such as less than or equal to ±5%, less than or equal to ±4%, less than or equal to ±3%, less than or equal to ±2%, less than or equal to ±1%, less than or equal to ±0.5%, less than or equal to ±0.1%, or less than or equal to ±0.05%. For example, two numerical values can be deemed to be "substantially" the same if a difference between the values is less than or equal to ±10% of an average of the values, such as less than or equal to ±5%, less than or equal to ±4%, less than or equal to ±3%, less than or equal to ±2%, less than or equal to ±1%, less than or equal to ±0.5%, less than or equal to ±0.1%, or less than or equal to ±0.05%.

Additionally, amounts, ratios, and other numerical values are sometimes presented herein in a range format. It is to be understood that such range format is used for convenience and brevity and should be understood flexibly to include numerical values explicitly specified as limits of a range, but also to include all individual numerical values or sub-ranges encompassed within that range as if each numerical value and sub-range is explicitly specified.

While the present disclosure has been described and illustrated with reference to specific embodiments thereof, these descriptions and illustrations do not limit the present disclosure. The illustrations may not be necessarily drawn to scale. There may be distinctions between the artistic renditions in the present disclosure and the actual apparatus due to manufacturing processes, tolerances and/or other reasons. There may be other embodiments of the present disclosure which are not specifically illustrated. The specification (other than the claims) and drawings are to be regarded as illustrative rather than restrictive. Modifications are possible within the scope of the claims appended hereto. While the techniques disclosed herein have been described with reference to particular operations performed in a particular order, it will be understood that these operations may be combined, sub-divided, or re-ordered to form an equivalent technique without departing from the teachings of the present disclosure. Accordingly, unless specifically indicated herein, the order and grouping of the operations are not limitations of the present disclosure. The scope of invention is defined by the appended claims.

**Claims**

1.  A computer-implemented method for monitoring and/or controlling a biopharmaceutical process, the method comprising:

    determining, by one or more processors, a query point (254) associated with scanning of the biopharmaceutical process by a spectroscopy system (106, 108);
    querying, by the one or more processors (120), an observation database (136) containing a plurality of observation data sets associated with past observations of biopharmaceutical processes, wherein each of the observation data sets includes spectral data and a corresponding actual analytical measurement, and wherein querying the observation database (136) includes selecting as training data, from among the plurality of observation data sets, observation data sets that satisfy one or more relevancy criteria with respect to the query point;
    calibrating, by the one or more processors and using the selected training data, a local model (132) specific to the biopharmaceutical process, the local model being a Gaussian process machine-learning model trained to predict analytical measurements based on spectral data inputs;
    predicting, by the one or more processors, an analytical measurement of the biopharmaceutical process, wherein predicting the analytical measurement of the biopharmaceutical process includes using the local model (132) to analyze spectral data that the spectroscopy system generated when scanning the biopharmaceutical process; and
    using the local model to determine a confidence indicator associated with the predicted analytical measurement of the biopharmaceutical process.

2.  The computer-implemented method of claim 1, wherein at least one of:

    a) the spectroscopy system is a Raman spectroscopy system (106, 108); or
    b) determining a query point includes determining the query point (254) based at least in part on a spectral scan vector, the spectral scan vector being generated by the spectroscopy system (106, 108) when scanning the biopharmaceutical process; and

    selecting as training data the observation data sets that satisfy one or more relevancy criteria with respect to the query point (254) includes comparing the spectral scan vector on which determination of the query point (254) was based to spectral scan vectors associated with the past observations of the biopharmaceutical processes.

3.  The computer-implemented method of claim 2, wherein:

determining a query point (254) further includes determining the query point (254) based on a sample number associated with the spectral scan vector; and

selecting as training data the observation data sets that satisfy one or more relevancy criteria with respect to the query point (254) includes (i) comparing the spectral scan vector on which determination of the query point (254) was based to spectral scan vectors associated with the past observations of the biopharmaceutical processes, and (ii) comparing the sample number associated with the query point (254) to sample numbers associated with the past observations of the biopharmaceutical processes.

4. The computer-implemented method of claim 3, wherein selecting as training data the observation data sets that satisfy one or more relevancy criteria with respect to the query point (254) includes:
selecting the most recent k observation data sets for inclusion in the training data.

5. The computer-implemented method of any one of claims 2 through 4, wherein at least one of:

a) predicting the analytical measurement of the biopharmaceutical process includes:
using the local model (132) to analyze the spectral scan vector on which determination of the query point (254) was based; or
b) selecting as training data the observation data sets that satisfy one or more relevancy criteria with respect to the query point (254) includes:

calculating distances between (i) the spectral scan vector on which determination of the query point (254) was based and (ii) the spectral scan vectors associated with the past observations of the biopharmaceutical processes; and
selecting as the training data any of the spectral scan vectors associated with the past observations that are within a threshold distance of the spectral scan vector on which determination of the query point (254) was based.

6. The computer-implemented method of any one of claims 1 through 5, wherein at least one of:

a) determining a query point (254) includes:
determining the query point (254) based at least in part on one or both of (i) a media profile associated with the biopharmaceutical process, and (ii) one or more operating conditions under which the biopharmaceutical process is analyzed;
b) calibrating a local model (132) specific to the biopharmaceutical process includes: calibrating a model that is a function of both spectral data and sample number of a given observation data set;
c) the method further comprising: controlling, by the one or more processors (120) and based at least in part on the predicted analytical measurement of the biopharmaceutical process, at least one parameter of the biopharmaceutical process;
d) the predicted analytical measurement of the biopharmaceutical process is a media component concentration, a media state, a viable cell density, a titer, a critical quality attribute, or a cell state;
e) the predicted analytical measurement of the biopharmaceutical process is a concentration of glucose, lactate, glutamate, glutamine, ammonia, amino acids, Na+, or K+;
f) the predicted analytical measurement of the biopharmaceutical process is pH, pCO2, pO2, temperature, or osmolality;
g) the method further comprising: obtaining, by an analytical instrument, an actual analytical measurement of the biopharmaceutical process; and
causing, by the one or more processors, (i) spectral data that the spectroscopy system generated when the actual analytical measurement was obtained, and (ii) the actual analytical measurement of the biopharmaceutical process, to be added to the observation database; or
h) the biopharmaceutical process is a cell culture process.

7. The computer-implemented method of claim 6, further comprising:

determining, by the one or more processors (120) that at least the predicted analytical measurement does not satisfy one or more model performance criteria,
wherein obtaining the actual analytical measurement is performed in response to determining that at least the predicted analytical measurement does not satisfy the one or more model performance criteria.

8. The computer-implemented method of claim 7, wherein determining that at least the predicted analytical measurement does not satisfy the one or more model performance criteria includes:

    generating a credibility interval associated with the predicted analytical measurement; and
    comparing the credibility interval to a pre-defined threshold.

9. A spectroscopy system for monitoring and/or controlling a biopharmaceutical process, the spectroscopy system comprising:

    one or more spectroscopy probes (108) collectively configured to (i) deliver source electromagnetic radiation to the biopharmaceutical process and (ii) collect electromagnetic radiation while the source electromagnetic radiation is delivered to the biopharmaceutical process;
    one or more memories collectively storing an observation database (136) containing a plurality of observation data sets associated with past observations of biopharmaceutical processes, wherein each of the observation data sets includes spectral data and a corresponding actual analytical measurement; and
    one or more processors (120) configured to determine a query point (254) associated with scanning of the biopharmaceutical process by the spectroscopy system,
    query the observation database, at least by selecting as training data, from among the plurality of observation data sets, observation data sets that satisfy one or more relevancy criteria with respect to the query point,
    calibrate, using the selected training data, a local model specific to the biopharmaceutical process, the local model being a Gaussian process machine-learning model trained to predict analytical measurements based on spectral data inputs, and
    predict an analytical measurement of the biopharmaceutical process, at least by using the local model (132, 260) to analyze spectral data that the spectroscopy system generated when scanning the biopharmaceutical process with the one or more spectroscopy probes; and
    using the local model to determine a confidence indicator associated with the predicted analytical measurement of the biopharmaceutical process.

10. The spectroscopy system of claim 9, wherein at least one of:

    a) the spectroscopy system is a Raman spectroscopy system (106, 108);
    b) the one or more processors (120) are configured to: determine the query point (254) based at least in part on a spectral scan vector, the spectral scan vector being generated by the spectroscopy system when scanning the biopharmaceutical process; and
    select the training data at least by comparing the spectral scan vector on which determination of the query point was based to spectral scan vectors associated with the past observations of the biopharmaceutical processes;
    c) the local model (132) is a function of both spectral data and sample number of a given observation data set;
    d) the one or more processors are further configured to: control, based at least in part on the predicted analytical measurement of the biopharmaceutical process, at least one parameter of the biopharmaceutical process;
    e) the predicted analytical measurement of the biopharmaceutical process is a media component concentration, a media state, a viable cell density, a titer, a critical quality attribute, or a cell state;
    f) the predicted analytical measurement of the biopharmaceutical process is a concentration of glucose, lactate, glutamate, glutamine, ammonia, amino acids, Na+, or K+;
    g) the predicted analytical measurement of the biopharmaceutical process is pH, pCO2, pO2, temperature, or osmolality;
    h) further comprising: an analytical instrument (104) configured to obtain an actual analytical measurement of the biopharmaceutical process, and wherein the one or more processors are further configured to cause (i) spectral data that the spectroscopy system generated when the actual analytical measurement was obtained, and (ii) the actual analytical measurement of the biopharmaceutical process, to be added to the observation database;
    i) the one or more processors are further configured to: determine that at least the predicted analytical measurement does not satisfy one or more model performance criteria; and
    obtain the actual analytical measurement from the analytical instrument in response to determining that at least the predicted analytical measurement does not satisfy the one or more model performance criteria;
    j) the one or more processors are configured to determine that at least the predicted analytical measurement does not satisfy the one or more model performance criteria at least by:

        generating a credibility interval associated with the predicted analytical measurement; and
        comparing the credibility interval to a pre-defined threshold; or

k) the biopharmaceutical process is a cell culture process.

11. The spectroscopy system of claim 10, wherein the one or more processors (120) are configured to:

determine the query point (254) based in part on a sample number associated with the spectral scan vector; and select as training data the observation data sets that satisfy one or more relevancy criteria with respect to the query point in part by (i) comparing the spectral scan vector on which determination of the query point was based to spectral scan vectors associated with the past observations of the biopharmaceutical processes, and (ii) comparing the sample number associated with the query point to sample numbers associated with the past observations of the biopharmaceutical processes.

12. The spectroscopy system of claim 11, wherein the one or more processors (120) are configured to select as training data the observation data sets that satisfy one or more relevancy criteria with respect to the query point (254) in part by: selecting the most recent k observation data sets for inclusion in the training data.

13. A non-transitory computer-readable medium storing instructions for monitoring and/or controlling a biopharmaceutical process, wherein the instructions, when executed by one or more processors, cause the one or more processors to perform the method of any one of claims 1 through 8.

14. A bioreactor system, comprising:
a bioreactor chamber (102) configured for containing a biopharmaceutical process; and a spectroscopy system (106, 108) as in any one of claims 9 through 12

15. The bioreactor system of claim 14 configured to produce a recombinant protein in a cell culture process.

## Patentansprüche

1. Computerimplementiertes Verfahren zum Überwachen und/oder Steuern eines biopharmazeutischen Prozesses, das Verfahren Folgendes umfassend:

Bestimmen, durch mindestens einen Prozessor, eines Abfragepunkts (254), der einem Abtasten des biopharmazeutischen Prozesses durch ein Spektroskopiesystem (106, 108) zugeordnet ist;
Abfragen, durch den mindestens einen Prozessor (120), einer Beobachtungsdatenbank (136), die eine Vielzahl von Beobachtungsdatensätzen enthält, die früheren Beobachtungen biopharmazeutischer Prozesse zugeordnet sind, wobei jeder der Beobachtungsdatensätze Spektraldaten und eine entsprechende tatsächliche analytische Messung umfasst und wobei Abfragen der Beobachtungsdatenbank (136) Auswählen von Beobachtungsdatensätzen, die mindestens ein Relevanzkriterium in Bezug auf den Abfragepunkt erfüllen, als Trainingsdaten aus der Vielzahl von Beobachtungsdatensätzen umfasst;
Kalibrieren, durch den mindestens einen Prozessor und durch Verwenden der ausgewählten Trainingsdaten, eines lokalen Modells (132), das für den biopharmazeutischen Prozess spezifisch ist, wobei das lokale Modell ein Gauß-Prozess-Maschinenlernmodell ist, das trainiert ist, um analytische Messungen auf Grundlage von Spektraldateneingaben vorherzusagen;
Vorhersagen, durch den mindestens einen Prozessor, einer analytischen Messung des biopharmazeutischen Prozesses, wobei Vorhersagen der analytischen Messung des biopharmazeutischen Prozesses Verwenden des lokalen Modells (132) zum Analysieren von Spektraldaten umfasst, die das Spektroskopiesystem beim Abtasten des biopharmazeutischen Prozesses erzeugt; und
Verwenden des lokalen Modells, um einen Konfidenzindikator zu bestimmen, welcher der vorhergesagten analytischen Messung des biopharmazeutischen Prozesses zugeordnet ist.

2. Computerimplementiertes Verfahren nach Anspruch 1, wobei mindestens eines des Folgenden gilt:

a) das Spektroskopiesystem ist ein Raman-Spektroskopiesystem (106, 108); oder
b) Bestimmen eines Abfragepunkts umfasst Bestimmen des Abfragepunkts (254) mindestens teilweise auf Grundlage eines Spektralabtastvektors, wobei der Spektralabtastvektor durch das Spektroskopiesystem (106, 108) beim Abtasten des biopharmazeutischen Prozesses erzeugt wird; und

Auswählen der Beobachtungsdatensätze, die mindestens ein Relevanzkriterium in Bezug auf den Abfragepunkt (254) erfüllen, als Trainingsdaten umfasst Vergleichen des Spektralabtastvektors, auf dem eine Bestimmung des Abfragepunkts (254) basiert, mit Spektralabtastvektoren, die den früheren Beobachtungen der biopharmazeutischen Prozesse zugeordnet sind.

3. Computerimplementiertes Verfahren nach Anspruch 2, wobei:

Bestimmen eines Abfragepunkts (254) weiterhin Bestimmen des Abfragepunkts (254) auf Grundlage eines Stichprobenumfangs umfasst, der dem Spektralabtastvektor zugeordnet ist; und
Auswählen der Beobachtungsdatensätze, die mindestens ein Relevanzkriterium in Bezug auf den Abfragepunkt (254) erfüllen, als Trainingsdaten (i) Vergleichen des Spektralabtastvektors, auf dem eine Bestimmung des Abfragepunkts (254) basiert, mit Spektralabtastvektoren, die den früheren Beobachtungen der biopharmazeutischen Prozesse zugeordnet sind, und (ii) Vergleichen des Stichprobenumfangs, der dem Abfragepunkt (254) zugeordnet ist, mit Stichprobenumfängen umfasst, die den früheren Beobachtungen der biopharmazeutischen Prozesse zugeordnet sind.

4. Computerimplementiertes Verfahren nach Anspruch 3, wobei Auswählen der Beobachtungsdatensätze, die mindestens ein Relevanzkriterium in Bezug auf den Abfragepunkt (254) erfüllen, als Trainingsdaten Folgendes umfasst: Auswählen der jüngsten k Beobachtungsdatensätze zur Einbeziehung in die Trainingsdaten.

5. Computerimplementiertes Verfahren nach einem der Ansprüche 2 bis 4, wobei mindestens eines des Folgenden gilt:

a) Vorhersagen der analytischen Messung des biopharmazeutischen Prozesses umfasst Folgendes:
Verwenden des lokalen Modells (132) zum Analysieren des Spektralabtastvektors, auf dem eine Bestimmung des Abfragepunkts (254) basiert; oder
b) Auswählen der Beobachtungsdatensätze, die mindestens ein Relevanzkriterium in Bezug auf den Abfragepunkt (254) erfüllen, als Trainingsdaten umfasst Folgendes:

Berechnen von Abständen zwischen (i) dem Spektralabtastvektor, auf dem eine Bestimmung des Abfragepunkts (254) basiert, und (ii) den Spektralabtastvektoren, die den früheren Beobachtungen der biopharmazeutischen Prozesse zugeordnet sind; und
Auswählen eines der Spektralabtastvektoren, die den früheren Beobachtungen zugeordnet sind, die innerhalb eines Schwellenabstands des Spektralabtastvektors liegen, auf dem eine Bestimmung des Abfragepunkts (254) basiert, als die Trainingsdaten.

6. Computerimplementiertes Verfahren nach einem der Ansprüche 1 bis 5, wobei mindestens eines des Folgenden gilt:

a) Bestimmen eines Abfragepunkts (254) umfasst Folgendes:
Bestimmen des Abfragepunkts (254) mindestens teilweise auf Grundlage eines oder beider von (i) einem Medienprofil, das dem biopharmazeutischen Prozess zugeordnet ist, und (ii) mindestens einer Betriebsbedingung, unter welcher der biopharmazeutische Prozess analysiert wird;
b) Kalibrieren eines lokalen Modells (132), das für den biopharmazeutischen Prozess spezifisch ist, umfasst Folgendes:
Kalibrieren eines Modells, das eine Funktion sowohl von Spektraldaten als auch eines Stichprobenumfangs eines gegebenen Beobachtungsdatensatzes ist;
c) das Verfahren umfasst weiterhin Folgendes: Steuern, durch den mindesten einen Prozessor (120) und mindestens teilweise auf Grundlage der vorhergesagten analytischen Messung des biopharmazeutischen Prozesses, mindestens eines Parameters des biopharmazeutischen Prozesses;
d) die vorhergesagte analytische Messung des biopharmazeutischen Prozesses ist eine Medienkomponentenkonzentration, ein Medienzustand, eine Dichte lebensfähiger Zellen, ein Titer, ein kritisches Qualitätsmerkmal oder ein Zellzustand;
e) die vorhergesagte analytische Messung des biopharmazeutischen Prozesses ist eine Konzentration von Glukose, Laktat, Glutamat, Glutamin, Ammoniak, Aminosäuren, Na+ oder K+;
f) die vorhergesagte analytische Messung des biopharmazeutischen Prozesses ist pH, pCO2, pO2, Temperatur oder Osmolalität;
g) das Verfahren umfasst weiterhin Folgendes:

Erlangen, durch ein analytisches Instrument, einer tatsächlichen analytischen Messung des biopharmazeutischen Prozesses; und

Bewirken, durch den mindestens einen Prozessor, (i) dass Spektraldaten, die das Spektroskopiesystem erzeugt, als die tatsächliche analytische Messung erlangt wurde, und (ii) die tatsächliche analytische Messung des biopharmazeutischen Prozesses zu der Beobachtungsdatenbank hinzugefügt werden; oder

h) der biopharmazeutische Prozess ist ein Zellkulturprozess.

7. Computerimplementiertes Verfahren nach Anspruch 6, weiterhin Folgendes umfassend:

Bestimmen, durch den mindestens einen Prozessor (120), dass mindestens die vorhergesagte analytische Messung mindestens ein Modellleistungskriterium nicht erfüllt, wobei Erlangen der tatsächlichen analytischen Messung als Reaktion auf Bestimmen, dass mindestens die vorhergesagte analytische Messung das mindestens eine Modellleistungskriterium nicht erfüllt, durchgeführt wird.

8. Computerimplementiertes Verfahren nach Anspruch 7, wobei Bestimmen, dass mindestens die vorhergesagte analytische Messung das mindestens eine Modellleistungskriterium nicht erfüllt, Folgendes umfasst:

Erzeugen eines Vertrauensintervalls, das der vorhergesagten analytischen Messung zugeordnet ist; und Vergleichen des Vertrauensintervalls mit einem vordefinierten Schwellenwert.

9. Spektroskopiesystem zum Überwachen und/oder Steuern eines biopharmazeutischen Prozesses, das Spektroskopiesystem Folgendes umfassend:

mindestens eine Spektroskopiesonde (108), die zusammen eingerichtet sind, um (i) elektromagnetische Quellenstrahlung an den biopharmazeutischen Prozess zu liefern und (ii) elektromagnetische Strahlung zu erfassen, während die elektromagnetische Quellenstrahlung an den biopharmazeutischen Prozess geliefert wird; mindestens einen Speicher, die zusammen eine Beobachtungsdatenbank (136) speichern, die eine Vielzahl von Beobachtungsdatensätzen enthält, die früheren Beobachtungen biopharmazeutischer Prozesse zugeordnet sind, wobei jeder der Beobachtungsdatensätze Spektraldaten und eine entsprechende tatsächliche analytische Messung umfasst; und mindestens einen Prozessor (120), der eingerichtet ist, um einen Abfragepunkt (254) zu bestimmen, der einem Abtasten des biopharmazeutischen Prozesses durch das Spektroskopiesystem zugeordnet ist, die Beobachtungsdatenbank mindestens durch Auswählen von Beobachtungsdatensätzen, die mindestens ein Relevanzkriterium in Bezug auf den Abfragepunkt erfüllen, als Trainingsdaten aus der Vielzahl von Beobachtungsdatensätzen abzufragen, unter Verwendung der ausgewählten Trainingsdaten, ein lokales Modell zu kalibrieren, das für den biopharmazeutischen Prozess spezifisch ist, wobei das lokale Modell ein Gauß-Prozess-Maschinenlernmodell ist, das trainiert ist, um analytische Messungen auf Grundlage von Spektraldateneingaben vorherzusagen, und eine analytische Messung des biopharmazeutischen Prozesses mindestens unter Verwendung des lokalen Modells (132, 260) zum Analysieren von Spektraldaten, die das Spektroskopiesystem beim Abtasten des biopharmazeutischen Prozesses mit der mindestens einen Spektroskopiesonde erzeugt, vorherzusagen; und das lokale Modell zu verwenden, um einen Konfidenzindikator zu bestimmen, welcher der vorhergesagten analytischen Messung des biopharmazeutischen Prozesses zugeordnet ist.

10. Spektroskopiesystem nach Anspruch 9, wobei mindestens eines des Folgenden gilt:

a) das Spektroskopiesystem ist ein Raman-Spektroskopiesystem (106, 108);
b) der mindestens eine Prozessor (120) ist eingerichtet, um: den Abfragepunkt (254) mindestens teilweise auf Grundlage eines Spektralabtastvektors zu bestimmen, wobei der Spektralabtastvektor durch das Spektroskopiesystem beim Abtasten des biopharmazeutischen Prozesses erzeugt wird; und die Trainingsdaten mindestens durch Vergleichen des Spektralabtastvektors, auf dem eine Bestimmung des Abfragepunkts basiert, mit Spektralabtastvektoren auszuwählen, die den früheren Beobachtungen der biopharmazeutischen Prozesse zugeordnet sind;
c) das lokale Modell (132) ist eine Funktion sowohl von Spektraldaten als auch eines Stichprobenumfangs eines gegebenen Beobachtungsdatensatzes;
d) der mindestens eine Prozessor ist weiterhin eingerichtet, um: mindestens teilweise auf Grundlage der vorhergesagten analytischen Messung des biopharmazeutischen Prozesses mindestens einen Parameter des biopharmazeutischen Prozesses zu steuern;

e) die vorhergesagte analytische Messung des biopharmazeutischen Prozesses ist eine Medienkomponentenkonzentration, ein Medienzustand, eine Dichte lebensfähiger Zellen, ein Titer, ein kritisches Qualitätsmerkmal oder ein Zellzustand;

f) die vorhergesagte analytische Messung des biopharmazeutischen Prozesses ist eine Konzentration von Glukose, Laktat, Glutamat, Glutamin, Ammoniak, Aminosäuren, Na+ oder K+;

g) die vorhergesagte analytische Messung des biopharmazeutischen Prozesses ist pH, pCO2, pO2, Temperatur oder Osmolalität;

h) weiterhin Folgendes umfassend: ein analytisches Instrument (104), das eingerichtet ist, um eine tatsächliche analytische Messung des biopharmazeutischen Prozesses zu erlangen, und wobei der mindestens eine Prozessor weiterhin eingerichtet ist, um zu bewirken, dass (i) Spektraldaten, die das Spektroskopiesystem erzeugt, als die tatsächliche analytische Messung erlangt wurde, und (ii) die tatsächliche analytische Messung des biopharmazeutischen Prozesses zu der Beobachtungsdatenbank hinzugefügt werden;

i) der mindestens eine Prozessor ist weiterhin eingerichtet, um: zu bestimmen, dass mindestens die vorhergesagte analytische Messung mindestens ein Modellleistungskriterium nicht erfüllt; und

die tatsächliche analytische Messung aus dem analytischen Instrument als Reaktion auf Bestimmen zu erlangen, dass mindestens die vorhergesagte analytische Messung das mindestens eine Modellleistungskriterium nicht erfüllt;

j) der mindestens eine Prozessor ist eingerichtet, um zu bestimmen, dass mindestens die vorhergesagte analytische Messung das mindestens eine Modellleistungskriterium nicht erfüllt, mindestens durch Folgendes:

Erzeugen eines Vertrauensintervalls, das der vorhergesagten analytischen Messung zugeordnet ist; und Vergleichen des Vertrauensintervalls mit einem vordefinierten Schwellenwert; oder

k) der biopharmazeutische Prozess ist ein Zellkulturprozess.

11. Spektroskopiesystem nach Anspruch 10, wobei der mindestens eine Prozessor (120) eingerichtet ist, um:

den Abfragepunkt (254) teilweise auf Grundlage eines Stichprobenumfangs zu bestimmen, der dem Spektralabtastvektor zugeordnet ist; und

die Beobachtungsdatensätze, die mindestens ein Relevanzkriterium in Bezug auf den Abfragepunkt erfüllen, als Trainingsdaten mindestens teilweise durch (i) Vergleichen des Spektralabtastvektors, auf dem eine Bestimmung des Abfragepunkts basiert, mit Spektralabtastvektoren, die den früheren Beobachtungen der biopharmazeutischen Prozesse zugeordnet sind, und (ii) Vergleichen des Stichprobenumfangs, der dem Abfragepunkt zugeordnet ist, mit Stichprobenumfängen umfasst, die den früheren Beobachtungen der biopharmazeutischen Prozesse zugeordnet sind, auszuwählen.

12. Spektroskopiesystem nach Anspruch 11, wobei der mindestens eine Prozessor (120) eingerichtet ist, um als Trainingsdaten die Beobachtungsdatensätze, die mindestens ein Relevanzkriterium in Bezug auf den Abfragepunkt (254) erfüllen, teilweise durch Folgendes auszuwählen:

Auswählen der jüngsten k Beobachtungsdatensätze zur Einbeziehung in die Trainingsdaten.

13. Nichtflüchtiges computerlesbares Medium, das Befehle zum Überwachen und/oder Steuern eines biopharmazeutischen Prozesses speichert, wobei die Befehle, wenn sie von mindestens einem Prozessor ausgeführt werden, bewirken, dass der mindestens eine Prozessor das Verfahren nach einem der Ansprüche 1 bis 8 durchführt.

14. Bioreaktorsystem, Folgendes umfassend:

eine Bioreaktorkammer (102), die zum Enthalten eines biopharmazeutischen Prozesses eingerichtet ist; und ein Spektroskopiesystem (106, 108) nach einem der Ansprüche 9 bis 12.

15. Bioreaktorsystem nach Anspruch 14, das eingerichtet ist, um ein rekombinantes Protein in einem Zellkulturprozess zu produzieren.

## Revendications

1. Procédé mis en œuvre par ordinateur pour surveiller et/ou contrôler un processus biopharmaceutique, le procédé comprenant :

la détermination, par un ou plusieurs processeurs, d'un point de requête (254) associé au balayage du processus biopharmaceutique par un système spectroscopique (106, 108) ;

l'interrogation, par le ou les processeurs (120), d'une base de données d'observation (136) contenant une pluralité d'ensembles de données d'observation associés à des observations passées de processus biopharmaceutiques, chacun des ensembles de données d'observation comportant des données spectrales et une mesure analytique réelle correspondante, et l'interrogation de la base de données d'observation (136) comportant la sélection comme données d'entraînement, parmi la pluralité d'ensembles de données d'observation, d'ensembles de données d'observation qui répondent à un ou plusieurs critères de pertinence par rapport au point de requête ;

l'étalonnage, par le ou les processeurs et au moyen des données d'entraînement sélectionnées, d'un modèle local (132) spécifique du processus biopharmaceutique, le modèle local étant un modèle d'apprentissage automatique à processus gaussiens entraîné à prédire des mesures analytiques sur la base d'entrées de données spectrales ;

la prédiction, par le ou les processeurs, d'une mesure analytique du processus biopharmaceutique, la prédiction de la mesure analytique du processus biopharmaceutique comportant l'utilisation du modèle local (132) pour analyser des données spectrales que le système spectroscopique a générées lors du balayage du processus biopharmaceutique ; et

l'utilisation du modèle local pour déterminer un indicateur de confiance associé à la mesure analytique prédite du processus biopharmaceutique.

2. Procédé mis en œuvre par ordinateur selon la revendication 1, dans lequel au moins une des situations suivantes prévaut :

a) le système spectroscopique est un système de spectroscopie Raman (106, 108) ; ou

b) la détermination d'un point de requête comporte la détermination du point de requête (254) au moins en partie sur la base d'un vecteur de balayage spectral, le vecteur de balayage spectral étant généré par le système spectroscopique (106, 108) lors du balayage du processus biopharmaceutique ; et

la sélection comme données d'entraînement des ensembles de données d'observation qui répondent à un ou plusieurs critères de pertinence par rapport au point de requête (254) comporte la comparaison du vecteur de balayage spectral sur lequel était basée la détermination du point de requête (254) à des vecteurs de balayage spectral associés aux observations passées des processus biopharmaceutiques.

3. Procédé mis en œuvre par ordinateur selon la revendication 2, dans lequel :

la détermination d'un point de requête (254) comporte en outre la détermination du point de requête (254) sur la base d'un numéro d'échantillon associé au vecteur de balayage spectral ; et

la sélection comme données d'entraînement des ensembles de données d'observation qui répondent à un ou plusieurs critères de pertinence par rapport au point de requête (254) comporte (i) la comparaison du vecteur de balayage spectral sur lequel était basée la détermination du point de requête (254) à des vecteurs de balayage spectral associés aux observations passées des processus biopharmaceutiques, et (ii) la comparaison du numéro d'échantillon associé au point de requête (254) à des numéros d'échantillon associés aux observations passées des processus biopharmaceutiques.

4. Procédé mis en œuvre par ordinateur selon la revendication 3, dans lequel la sélection comme données d'entraînement des ensembles de données d'observation qui répondent à un ou plusieurs critères de pertinence par rapport au point de requête (254) comporte :

la sélection des k ensembles de données d'observation les plus récents pour inclusion dans les données d'entraînement.

5. Procédé mis en œuvre par ordinateur selon l'une quelconque des revendications 2 à 4, dans lequel au moins une des situations suivantes prévaut :

a) la prédiction de la mesure analytique du processus biopharmaceutique comporte :

l'utilisation du modèle local (132) pour analyser le vecteur de balayage spectral sur lequel était basée la détermination du point de requête (254) ; ou

b) la sélection comme données d'entraînement des ensembles de données d'observation qui répondent à un ou plusieurs critères de pertinence par rapport au point de requête (254) comporte :

le calcul de distances entre (i) le vecteur de balayage spectral sur lequel était basée la détermination du point de requête (254) et (ii) les vecteurs de balayage spectral associés aux observations passées des processus biopharmaceutiques ; et

la sélection comme données d'entraînement de tous les vecteurs de balayage spectral associés aux observations passées qui se situent à moins d'une distance seuil du vecteur de balayage spectral sur lequel était basée la détermination du point de requête (254).

**6.** Procédé mis en œuvre par ordinateur selon l'une quelconque des revendications 1 à 5, dans lequel au moins une des situations suivantes prévaut :

a) la détermination d'un point de requête (254) comporte :
la détermination du point de requête (254) au moins en partie sur la base (i) d'un profil de milieu associé au processus biopharmaceutique, et/ou (ii) d'une ou plusieurs conditions opérationnelles dans lesquelles le processus biopharmaceutique est analysé ;
b) l'étalonnage d'un modèle local (132) spécifique du processus biopharmaceutique comporte :
l'étalonnage d'un modèle qui est fonction à la fois de données spectrales et du numéro d'échantillon d'un ensemble donné de données d'observation ;
c) le procédé comprenant en outre : le contrôle, par le ou les processeurs (120) et au moins en partie sur la base de la mesure analytique prédite du processus biopharmaceutique, d'au moins un paramètre du processus biopharmaceutique ;
d) la mesure analytique prédite du processus biopharmaceutique est une concentration d'un constituant du milieu, un état du milieu, une densité de cellules viables, un titre, un attribut de qualité critique, ou un état cellulaire ;
e) la mesure analytique prédite du processus biopharmaceutique est une concentration de glucose, de lactate, de glutamate, de glutamine, d'ammoniac, d'acides aminés, de Na+, ou de K+ ;
f) la mesure analytique prédite du processus biopharmaceutique est le pH, le pCO2, le pO2, la température, ou l'osmolalité ;
g) le procédé comprenant en outre : l'obtention, par un instrument analytique, d'une mesure analytique réelle du processus biopharmaceutique ; et
l'induction, par le ou les processeurs, de l'ajout (i) de données spectrales que le système spectroscopique a générées quand la mesure analytique réelle a été obtenue, et (ii) de la mesure analytique réelle du processus biopharmaceutique, à la base de données d'observation ; ou
h) le processus biopharmaceutique est un processus de culture cellulaire.

**7.** Procédé mis en œuvre par ordinateur selon la revendication 6, comprenant en outre :

la détermination, par le ou les processeurs (120), qu'au moins la mesure analytique prédite ne répond pas à un ou plusieurs critères de performance du modèle,
dans lequel l'obtention de la mesure analytique réelle est effectuée en réponse à la détermination qu'au moins la mesure analytique prédite ne répond pas au(x) critère(s) de performance du modèle.

**8.** Procédé mis en œuvre par ordinateur selon la revendication 7, dans lequel la détermination qu'au moins la mesure analytique prédite ne répond pas au(x) critère(s) de performance du modèle comporte :

la génération d'un intervalle de crédibilité associé à la mesure analytique prédite ; et
la comparaison de l'intervalle de crédibilité à un seuil prédéfini.

**9.** Système spectroscopique destiné à surveiller et/ou contrôler un processus biopharmaceutique, le système spectroscopique comprenant :

une ou plusieurs sondes spectroscopiques (108) collectivement conçues pour (i) délivrer un rayonnement électromagnétique source au processus biopharmaceutique et (ii) collecter un rayonnement électromagnétique pendant que le rayonnement électromagnétique source est délivré au processus biopharmaceutique ;
une ou plusieurs mémoires stockant collectivement une base de données d'observation (136) contenant une pluralité d'ensembles de données d'observation associés à des observations passées de processus biopharmaceutiques, chacun des ensembles de données d'observation comportant des données spectrales et une mesure analytique réelle correspondante ; et
un ou plusieurs processeurs (120) conçus pour déterminer un point de requête (254) associé au balayage du processus biopharmaceutique par le système spectroscopique,

interroger la base de données d'observation, au moins en sélectionnant comme données d'entraînement, parmi la pluralité d'ensembles de données d'observation, des ensembles de données d'observation qui répondent à un ou plusieurs critères de pertinence par rapport au point de requête,

étalonner, en utilisant les données d'entraînement sélectionnées, un modèle local spécifique du processus biopharmaceutique, le modèle local étant un modèle d'apprentissage automatique à processus gaussiens entraîné à prédire des mesures analytiques sur la base d'entrées de données spectrales, et

prédire une mesure analytique du processus biopharmaceutique, au moins en utilisant le modèle local (132, 260) pour analyser des données spectrales que le système spectroscopique a générées lors du balayage du processus biopharmaceutique avec la ou les sondes spectroscopiques ; et

utiliser le modèle local pour déterminer un indicateur de confiance associé à la mesure analytique prédite du processus biopharmaceutique.

10. Système spectroscopique selon la revendication 9, dans lequel au moins une des situations suivantes prévaut :

a) le système spectroscopique est un système de spectroscopie Raman (106, 108) ;

b) le ou les processeurs (120) sont conçus pour :

déterminer le point de requête (254) au moins en partie sur la base d'un vecteur de balayage spectral, le vecteur de balayage spectral étant généré par le système spectroscopique lors du balayage du processus biopharmaceutique ; et

sélectionner les données d'entraînement au moins en comparant le vecteur de balayage spectral sur lequel était basée la détermination du point de requête à des vecteurs de balayage spectral associés aux observations passées des processus biopharmaceutiques ;

c) le modèle local (132) est fonction à la fois de données spectrales et du numéro d'échantillon d'un ensemble donné de données d'observation ;

d) le ou les processeurs sont en outre conçus pour : contrôler, au moins en partie sur la base de la mesure analytique prédite du processus biopharmaceutique, au moins un paramètre du processus biopharmaceutique ;

e) la mesure analytique prédite du processus biopharmaceutique est une concentration d'un constituant du milieu, un état du milieu, une densité de cellules viables, un titre, un attribut de qualité critique, ou un état cellulaire ;

f) la mesure analytique prédite du processus biopharmaceutique est une concentration de glucose, de lactate, de glutamate, de glutamine, d'ammoniac, d'acides aminés, de Na+, ou de K+ ;

g) la mesure analytique prédite du processus biopharmaceutique est le pH, le pCO2, le pO2, la température, ou l'osmolalité ;

h) comprenant en outre : un instrument analytique (104) conçu pour obtenir une mesure analytique réelle du processus biopharmaceutique, et dans lequel le ou les processeurs sont en outre conçus pour induire l'ajout (i) de données spectrales que le système spectroscopique a générées quand la mesure analytique réelle a été obtenue, et (ii) de la mesure analytique réelle du processus biopharmaceutique, à la base de données d'observation ;

i) le ou les processeurs sont en outre conçus pour :

déterminer qu'au moins la mesure analytique prédite ne répond pas à un ou plusieurs critères de performance du modèle ; et

obtenir la mesure analytique réelle auprès de l'instrument analytique en réponse à la détermination qu'au moins la mesure analytique prédite ne répond pas au(x) critère(s) de performance du modèle ;

j) le ou les processeurs sont conçus pour déterminer qu'au moins la mesure analytique prédite ne répond pas au(x) critère(s) de performance de modèle au moins :

en générant un intervalle de crédibilité associé à la mesure analytique prédite ; et

en comparant l'intervalle de crédibilité à un seuil prédéfini ; ou

k) le processus biopharmaceutique est un processus de culture cellulaire.

11. Système spectroscopique selon la revendication 10, dans lequel le ou les processeurs (120) sont conçus pour :

déterminer le point de requête (254) au moins en partie sur la base d'un numéro d'échantillon associé au vecteur de balayage spectral ; et

sélectionner comme données d'entraînement les ensembles de données d'observation qui répondent à un ou plusieurs critères de pertinence par rapport au point de requête en partie (i) en comparant le vecteur de balayage spectral sur lequel était basée la détermination du point de requête à des vecteurs de balayage spectral associés aux observations passées des processus biopharmaceutiques, et (ii) en comparant le numéro d'échantillon associé au point de requête à des numéros d'échantillon associés aux observations passées des processus biopharmaceutiques.

12. Système spectroscopique selon la revendication 11, dans lequel le ou les processeurs (120) sont conçus pour sélectionner comme données d'entraînement les ensembles de données d'observation qui répondent à un ou plusieurs critères de pertinence par rapport au point de requête (254) en partie :

en sélectionnant les k ensembles de données d'observation les plus récents pour inclusion dans les données d'entraînement.

13. Support non transitoire lisible par ordinateur stockant des instructions destinées à surveiller et/ou contrôler un processus biopharmaceutique, les instructions, lors de leur exécution par un ou plusieurs processeurs, conduisant le ou les processeurs à effectuer le procédé de l'une quelconque des revendications 1 à 8.

14. Système de bioréacteur, comprenant :

une chambre de bioréacteur (102) conçue pour contenir un processus biopharmaceutique ; et un système spectroscopique (106, 108) selon l'une quelconque des revendications 9 à 12.

15. Système de bioréacteur selon la revendication 14 conçu pour produire une protéine recombinante dans un processus de culture cellulaire.

FIG. 1

FIG. 2

200

**FIG. 3**

Legend:
- Uncertainty
- JITL Prediction
- Actual (○)
- Set Point (– –)

GLUCOSE (g/L) axis: 14, 12, 10, 8, 6, 4, 2, 0, -2, -4

TIME (date) axis: 12/06/17, 12/07/17, 12/08/17, 12/09/17, 12/10/17, 12/11/17, 12/12/17, 12/13/17, 12/14/17

208, 206, 204, 202

250

```
256                      254                    252
 ┌─────────────────┐    ┌──────────────┐      ┌──────────────┐
 │ Global Data Set │◄───│ Query Point  │◄─────│ Spectral Data│
 └─────────────────┘    └──────────────┘      └──────────────┘
         │                                            ▲
         ▼                                            │
258                                                   │
 ┌─────────────────┐                                  │
 │  Local Data Set │                                  │
 └─────────────────┘                                  │
         │                                            │
         ▼                                            │
260                                                   │
 ┌─────────────────┐                                  │
 │   Local Model   │                                  │
 └─────────────────┘                                  │
         │                                            │
         ▼                                            │
262                                                   │
 ┌─────────────────┐                                  │
 │ Predicted Output│                                  │
 └─────────────────┘                                  │
         │                                            │
         └────────────────────────────────────────────
         │
         ▼
```

**FIG. 4**

FIG. 5

350

**FIG. 6**

400

DETERMINE QUERY POINT ASSOCIATED WITH
SCANNING OF BIOPHARMACEUTICAL PROCESS
BY SPECTROSCOPY SYSTEM — 402

QUERY OBSERVATION DATABASE CONTAINING
OBSERVATION DATA SETS ASSOCIATED WITH
PAST OBSERVATIONS OF BIOPHARMACEUTICAL
PROCESSES, INCLUDING SELECTING AS
TRAINING DATA OBSERVATION DATA SETS THAT
SATISFY RELEVANCY CRITERIA WITH RESPECT
TO QUERY POINT — 404

USE SELECTED TRAINING DATA TO
CALIBRATE LOCAL MODEL SPECIFIC TO
BIOPHARMACEUTICAL PROCESS — 406

PREDICT ANALYTICAL MEASUREMENT OF
BIOPHARMACEUTICAL PROCESS USING
LOCAL MODEL — 408

# FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62749359
- US 62833044
- US 62864565
- US 2018291329 A1
- US 7741465 B
- US 6319494 B
- US 6660843 B
- US 7138370 B
- US 7511012 B
- WO 9954440 A
- WO 2005040220 A
- WO 2008119567 A
- US 20140302037 A
- US 20140308285 A
- WO 2014151910 A
- WO 2015048272 A
- WO 2013128027 A
- WO 2014140358 A
- WO 2017134140 A

**Non-patent literature cited in the description**

- **MEHDIZAHEH et al.** *Biotechnolo. Prog.*, 2015, vol. 31 (4), 1004-1013
- **WEBSTER et al.** *Biotechnol. Prog.*, 2018, vol. 34 (3), 730-737
- **SU QING-LIN et al.** Just-in-Time-Learning based Extended Prediction Self-Adaptive Control for batch processes. *Journal of Process Control*, 10 May 2016, vol. 43, 1-9
- A modified recursive locally weighted NIR modelling for fermentation process. **CHEN LINGYI et al.** 2017 6th International Symposium on Advanced Control of Industrial Processes (ADCONIP). IEEE, 28 May 2017, 559-564
- **YI LIU et al.** Just-in-Time Kernel Learning with Adaptive Parameter Selection for Soft Sensor Modeling of Batch Processes. *Industrial & Engineering Chemistry Research*, 08 March 2012, vol. 51 (11), 4313-4327
- **ESHHAR et al.** *Cancer Immunol Immunotherapy*, 1997, vol. 45, 131-136